(19) Europäisches Patentamt
European Patent Office
Office européen des brevets

(11) Veröffentlichungsnummer: **0 320 753**
**A2**

(12) # EUROPÄISCHE PATENTANMELDUNG

(21) Anmeldenummer: **88120325.1**

(22) Anmeldetag: **06.12.88**

(51) Int. Cl.4: **C07D 403/06 , C07D 401/14 , C07D 401/06 , C07D 453/06 , C07D 209/52 , C07D 209/70 , C07D 207/16 , C07D 207/08 , C07D 295/18 , A61K 31/40 , A61K 31/435**

Patentansprüche für folgende Vertragsstaaten: ES + GR.

(30) Priorität: **15.12.87 DE 3742431**

(43) Veröffentlichungstag der Anmeldung: **21.06.89 Patentblatt 89/25**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Henning, Rainer, Dr.**
**Rotenhofstrasse 31**
**D-6234 Hattersheim am Main(DE)**
Erfinder: **Urbach, Hansjörg, Dr.**
**Le Lavandoustrasse 41**
**D-6242 Kronberg/Taunus(DE)**
Erfinder: **Hock, Franz, Dr.**
**Altstadt 19**
**D-6110 Dieburg(DE)**

(54) **Neue Derivate cyclischer Aminosäuren, Verfahren zu ihrer Herstellung, sie enthaltende Mittel und deren Verwendung.**

(57) Die Erfindung betrifft Derivate cyclischer Aminosäuren der allgemeinen Formel

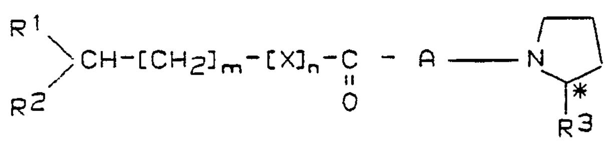

(I)

in welcher
$R^1$ Wasserstoff, Alkyl, Alkenyl oder (subst.) Aryl bedeutet, $R^2$ Wasserstoff, Alkyl, Hydroxy, Alkoxy, (subst.) Aryl, (subst.) Aryloxy oder (subst.) Aroyl ist, oder $R^1$ und $R^2$ zusammen für (subst.) Benzyliden oder $R^1$ und $R^2$ zusammen mit dem sie tragenden C-Atom für (subst.) Cycloalkyl stehen, $R^3$ Wasserstoff, Hydroxymethyl, Formyl, (subst.) Alkenyl, (subst.) Carboxylatocarbonyl, (subst.) Carbamoylatocarbonyl oder (subst.) Trifluormethylcarbonyl bedeutet, A für eine cyclische Aminosäure steht, X Sauerstoff, Imino oder N-Alkylimino bedeutet, m = 0-5 ist und n = 0 oder 1 ist, Verfahren zu ihrer Herstellung, sie enthaltende Mittel sowie deren Verwendung.

EP 0 320 753 A2

# Neue Derivate cyclischer Aminosäuren, Verfahren zu ihrer Herstellung, sie enthaltende Mittel und deren Verwendung

Die Erfindung betrifft neue, substituierte Prolylpyrrolidine mit Prolyl-Endopeptidase-hemmender Wirkung sowie Verfahren zu deren Herstellung.

In EP-A 232 849 werden u.a. Alkylcarbonyl-prolylpyrrolidin-Derivate mit unsubstituiertem Prolyl-Rest beschrieben.

Die dieser Erfindung zugrundeliegende Aufgabe, neue, potente Prolyl-Endopeptidase-Hemmer zu finden, wird durch die neuen Derivate cyclischer Aminosäuren der allgemeinen Formel I gelöst.

Verbindungen der allgemeinen Formel I

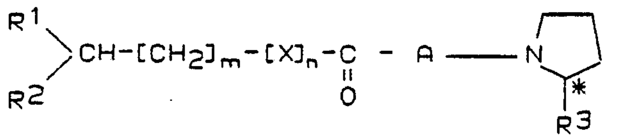

(I)

in welcher

$R^1$ Wasserstoff; $(C_1\text{-}C_{20})$-Alkyl; $(C_3\text{-}C_{20})$-Alkenyl; $(C_6\text{-}C_{12})$-Aryl, das gegebenenfalls durch einen, zwei oder drei gleiche oder verschiedene Reste aus der Reihe $(C_1\text{-}C_4)$-Alkyl, $(C_1\text{-}C_4)$-Alkoxy, Halogen, Nitro, Hydroxy, Amino, $(C_1\text{-}C_4)$-Alkylamino und Di-$(C_1\text{-}C_4)$-alkylamino, oder ein ($C_1$ oder $C_2$)-Alkylendioxy substituiert ist, bedeutet;

$R^2$ Wasserstoff; $(C_1\text{-}C_6)$-Alkyl; $(C_6\text{-}C_{12})$-Aryl; $(C_6\text{-}C_{12})$-Aryloxy; $(C_7\text{-}C_{13})$-Aroyl; Hydroxy oder $(C_1\text{-}C_4)$-Alkoxy bedeutet, wobei Aryl, Aryloxy und Aroyl jeweils gegebenenfalls durch einen, zwei oder drei gleiche oder verschiedene Reste aus der Reihe $(C_1\text{-}C_4)$-Alkyl, Phenyl-$(C_1\text{-}C_3)$-alkyl, $(C_1\text{-}C_4)$-Alkenyl, Phenyl-$(C_2\text{-}C_4)$-alkenyl, $(C_1\text{-}C_4)$-Alkoxy, $(C_1\text{-}C_4)$-Alkenyloxy, Phenyl-$(C_1\text{-}C_3)$- alkoxy, Halogen, Nitro, Hydroxy, Amino, $(C_1\text{-}C_4)$-Alkylamino und Di-$(C_1\text{-}C_4)$-alkylamino, oder ein ($C_1$ oder $C_2$)-Alkylendioxy substituiert sind, oder

$R^1$ und $R^2$ zusammen für Benzyliden stehen, worin der Phenylring gegebenenfalls durch einen, zwei oder drei gleiche oder verschiedene Reste aus der Reihe $(C_1\text{-}C_4)$-Alkyl, $(C_1\text{-}C_4)$-Alkoxy, Halogen, Nitro, Hydroxy, Amino, $(C_1\text{-}C_4)$-Alkylamino und Di-$(C_1\text{-}C_4)$-alkylamino, oder ein ($C_1$ oder $C_2$)-Alkylendioxy substituiert ist, oder $R^1$ und $R^2$ zusammen mit dem sie tragenden C-Atom für $(C_3\text{-}C_6)$-Cycloalkyl stehen, das gegebenenfalls durch Phenyl substituiert ist, bedeuten;

A einen Rest aus der Gruppe

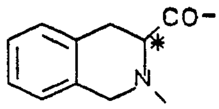 ; 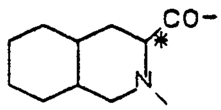 ; 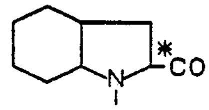 ; 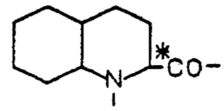 ;

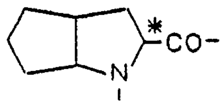 ; 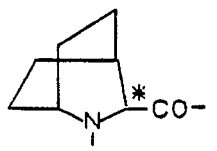 ; 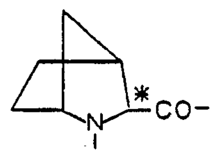 ; 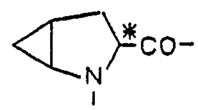 ;

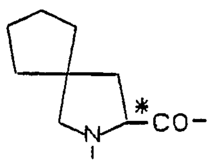 ; 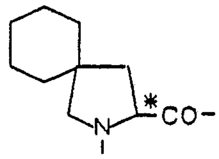 ; 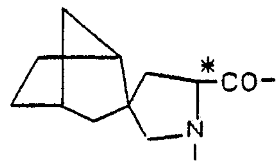 ;

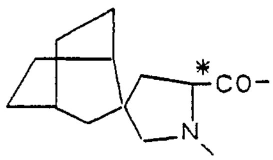 ; 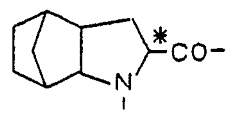 ; 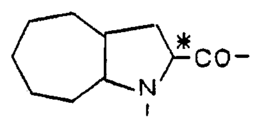 ;

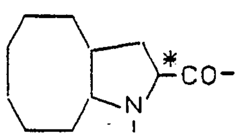 ; 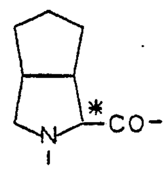 ; 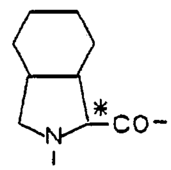 ;

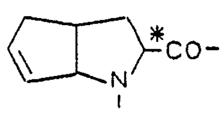 ; 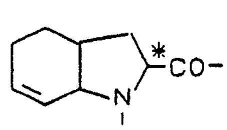 ; 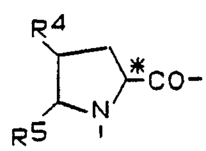 ;

bedeutet,
wobei $R^4$ und $R^5$ gleich oder verschieden sind und unabhängig voneinander Wasserstoff; $(C_1$-$C_6)$-Alkyl; $(C_5$-$C_8)$-Cycloalkyl; Phenyl oder Phenyl-$(C_1$-$C_4)$-alkyl, die jeweils gegebenenfalls durch $(C_1$-$C_4)$-Alkyl, $(C_1$-$C_4)$-Alkoxy oder Halogen mono- oder disubstituiert sein können, wobei jedoch höchstens einer der Reste $R^4$ oder $R^5$ Wasserstoff bedeuten kann; und
$R^3$ einen Rest aus der Gruppe Wasserstoff; Hydroxymethyl; Formyl;

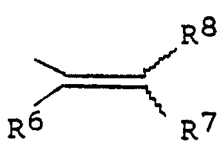 ; 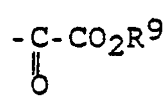 ; 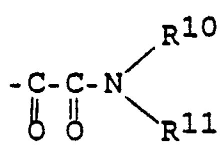 ;

$-CO-CF_3$; $-CO-CF_2-CO_2R^9$; $-CO-CF_2-R^{10}$ und

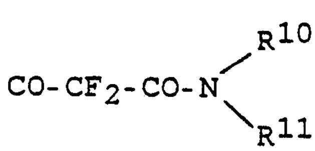 bedeuten, wobei

$R^6$ Wasserstoff; $(C_1-C_6)$-Alkyl oder $(C_6-C_{12})$-Aryl, das gegebenenfalls durch einen oder zwei gleiche oder verschiedene Reste aus der Reihe $(C_1-C_4)$-Alkyl, ($C_1$ oder $C_2$)-Alkoxy und Halogen, oder ein ($C_1$ oder $C_2$)-Alkylendioxy substituiert ist, bedeutet;

$R^7$ Wasserstoff; $(C_1-C_6)$-Alkyl; Cyano; $(C_7-C_{13})$-Aroyl, das gegebenenfalls durch einen oder zwei gleiche oder verschiedene Reste aus der Reihe $(C_1-C_4)$-Alkyl, ($C_1$ oder $C_2$)-Alkoxy, Halogen und Nitro, oder ein ($C_1$ oder $C_2$)-Alkylendioxy monosubstituiert ist; $(C_1-C_6)$-Alkoxycarbonyl oder $(C_1-C_8)$-Alkanoyl bedeutet;

$R^8$ Cyano; $(C_7-C_{13})$-Aroyl oder $(C_6-C_{12})$-Aryl-$(C_2-C_4)$-alkanoyl bedeutet, wobei jeweils Aryl durch einen, zwei oder drei gleiche oder verschiedene Reste aus der Reihe $(C_1-C_4)$-Alkyl, $(C_1-C_4)$-Alkoxy, Halogen, Nitro und Hydroxy oder ein ($C_1$ oder $C_2$)-Alkylendioxy substituiert sein kann; oder $(C_1-C_6)$-Alkanoyl; $(C_1-C_6)$-Alkoxycarbonyl; Benzyloxycarbonyl oder einen Rest der Formel

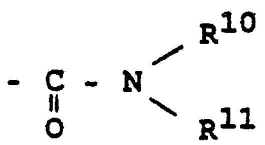

bedeutet;

$R^9$ Wasserstoff; $(C_1-C_6)$-Alkyl; Phenyl-$(C_1-C_4)$-alkyl oder Diphenyl-$(C_1-C_4)$-alkyl bedeutet;

$R^{10}$ und $R^{11}$ gleich oder verschieden sind und Wasserstoff; $(C_1-C_8)$-Alkyl; $(C_6-C_{12})$-Aryl oder $(C_6-C_{12})$-Aryl-$(C_1-C_8)$-alkyl, worin jeweils Aryl gegebenenfalls durch einen, zwei oder drei gleiche oder verschiedene Reste aus der Reihe $(C_1-C_4)$-Alkyl, $(C_1-C_4)$-Alkoxy, Halogen und Hydroxy oder ein ($C_1$ oder $C_2$)-Alkylendioxy substituiert ist; oder $(C_5-C_9)$-Cycloalkyl bedeuten; oder

$R^{10}$ und $R^{11}$ zusammen mit dem diese tragenden N-Atom einen 5- bis 10-gliedrigen heterocyclischen Ring bilden, der als Ringatome 2-9 Kohlenstoffatome und gegebenenfalls 1 oder 2 weitere gleiche oder verschiedene Heteroatome aus der Reihe Sauerstoff, Schwefel und Stickstoff enthält und der gegebenenfalls durch einen Rest aus der Reihe $(C_1-C_8)$-Alkyl, Phenyl, Phenyl-$(C_1-C_4)$-alkyl, Benzoyl, Phenyl-$(C_2-C_4)$-alkanoyl, 2- oder 3-Furoyl, 2-, 3- oder 4-Pyridyl und 2-, 4- oder 5-Pyrimidinyl substituiert ist, worin Phenyl, Phenylalkyl, Benzoyl und Phenylalkanoyl ihrerseits jeweils im Phenylrest durch einen, zwei oder drei gleiche oder verschiedene Reste aus der Reihe $(C_1-C_4)$-Alkyl, $(C_1-C_4)$-Alkoxy, Halogen, Hydroxy, Cyano und Nitro, oder ein ($C_1$ oder $C_2$)-Alkylendioxy substituiert sein können;

X Sauerstoff, Imino oder N-$(C_1-C_8)$-Alkylimino bedeutet;

m = 0, 1, 2, 3, 4 oder 5 ist; und

n = 0 oder 1 ist;

sowie deren physiologisch verträgliche Salze, falls solche gebildet werden können.

Alkyl kann geradkettig oder verzweigt sein und ist beispielsweise Methyl, Ethyl, Propyl, Isopropyl, Butyl, Isobutyl, sec.-Butyl, tert.-Butyl, Pentyl, Isopentyl, Neopentyl, sec.-Pentyl, tert.-Pentyl, Hexyl, Isohexyl, Heptyl oder Octyl. Entsprechendes gilt für davon abgeleitete Reste von Alkoxy, Alkylamino, Dialkylamino, Alkanoyl, Alkoxycarbonyl und Aralkyl.

Aryl ist beispielsweise Phenyl, α- oder β-Naphthyl, 2-, 3-oder 4-Biphenylyl; bevorzugt ist Phenyl. Entsprechendes gilt für davon abgeleitete Reste wie Aryloxy, Aralkyl, Aryl, Aroyl und Arylalkanoyl.

Halogen ist Fluor, Chlor, Brom oder Jod; bevorzugt sind Fluor, Chlor und Brom.

$R^{10}$ und $R^{11}$ können zusammen mit dem diese tragenden N-Atom einen 5- bis 10-gliedrigen, vorzugsweise 5- bis 7-gliedrigen heterocyclischen Ring bilden, der als Ringatome 2-9 Kohlenstoffatome und gegebenenfalls 1 oder 2 weitere gleiche oder verschiedene Heteroatome aus der Reihe Sauerstoff, Schwefel und Stickstoff enthält und der gegebenenfalls in der oben angegebenen Weise monosubstituiert ist. Dieser Heterocyclenrest kann gesättigt oder ungesättigt, mono- oder bicyclisch sein.

Bevorzugt sind jedoch monocyclische gesättigte Reste, von z.B. Piperidino, Pyrrolidino, Hexahydro-1-Azepinyl, Morpholino, Piperazino oder Homopiperazino.

Verbindungen der Formel I besitzen chirale C-Atome. Sowohl die R- als auch die S-Konfigurationen an allen Asymmetriezentren sind Gegenstand der Erfindung. Die Verbindungen der Formel I können daher als optische Isomere, als Diasteromere, als Racemate oder als Gemische derselben vorliegen. Bevorzugt sind jedoch die Verbindungen der Formel I, in denen die mit einem Stern (*) markierten C-Atome S-Konfiguration aufweisen.

Als Salze kommen insbesondere Alkali- oder Erdalkalisalze, Salze mit physiologisch verträglichen Aminen und Salze mit anorganischen oder organischen Säuren wie z.B. HCl, HBr, $H_2SO_4$, Maleinsäure, Fumarsäure in Frage.

Bevorzugt sind Verbindungen der Formel I, in welcher

$R^1$ Wasserstoff; $(C_1-C_{18})$-Alkyl; $(C_3-C_{18})$-Alkenyl oder Phenyl, das gegebenenfalls durch einen oder zwei gleiche oder verschiedene Reste aus der Reihe Methyl, Ethyl, Methoxy, Fluor, Chlor, Brom, Nitro, Hydroxy, Amino, Methylamino und Dimethylamino, oder drei Methoxy, oder ein Methylendioxy substituiert ist, bedeutet;

$R^2$ Wasserstoff; Phenyl; Phenoxy; Benzoyl; Hydroxy oder Methoxy bedeutet, wobei Phenyl, Phenoxy und Benzoyl jeweils gegebenenfalls durch einen oder zwei gleiche oder verschiedene Reste aus der Reihe Methyl, Ethyl, Benzyl, Phenethyl, Styryl, Fluor, Chlor, Brom, Nitro, Hydroxy, Amino, Methylamino und Dimethylamino, oder ein Methylendioxy substituiert sind; oder

$R^1$ und $R^2$ zusammen für Benzyliden stehen, worin der Phenylrest gegebenenfalls durch einen oder zwei gleiche oder verschiedene Reste aus der Reihe Methyl, Ethyl, Methoxy, Fluor, Chlor, Brom und Hydroxy, oder Methoxy, oder ein Methylendioxy substituiert ist oder $R^1$ und $R^2$ zusammen mit dem sie tragenden C-Atom für Cyclopropyl stehen, das gegebenenfalls durch Phenyl substituiert ist;

$R^6$ Wasserstoff; $(C_1-C_4)$-Alkyl; Phenyl; o-, m- oder p-Methoxyphenyl; 2,3-, 2,4-, 2,5-, 2,6-, 3,4- oder 3,5-Dimethoxyphenyl; o-, m- oder p-Tolyl; 2,3-, 2,4-, 2,5-, 2,6-, 3,4- oder 3,5-Xylyl; o-, m- oder p-Fluorphenyl; o-, m- oder p-Chlorphenyl oder 2,3- oder 3,4-Methylendioxyphenyl bedeutet;

$R^7$ Wasserstoff; $(C_1-C_4)$-Alkyl; Cyano; Benzoyl, das gegebenenfalls durch einen oder zwei gleiche oder verschiedene Reste aus der Reihe Methyl, Methoxy, Fluor, Chlor, Brom und Nitro, oder ein Methylendioxy substituiert ist; $(C_1-C_4)$-Alkoxycarbonyl oder $(C_1-C_5)$-Alkanoyl bedeutet;

$R^8$ Cyano; Benzoyl, das gegebenenfalls durch einen oder zwei gleiche oder verschiedene Reste aus der Reihe Methyl, Methoxy, Fluor, Chlor und Brom, drei Methoxy, oder ein Methylendioxy substituiert ist; $(C_1-C_4)$-Alkanoyl; $(C_1-C_4)$-Alkoxycarbonyl oder einen Rest der Formel

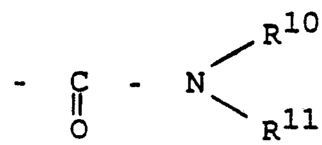

bedeutet;

$R^9$ $(C_1-C_4)$-Alkyl; Benzyl; Phenethyl oder Benzhydryl bedeutet;

$R^{10}$ und $R^{11}$ gleich oder verschieden sind und Wasserstoff; $(C_1-C_6)$-Alkyl; Phenyl oder Phenyl-$(C_1-C_4)$-alkyl, worin der Phenylrest jeweils durch einen oder zwei gleiche oder verschiedene Reste aus der Reihe Methyl, Ethyl, Methoxy, Fluor, Chlor, Brom und Hydroxy, drei Methoxy oder ein Methylendioxy substituiert sein kann; Cyclopentyl; Cyclohexyl oder Cycloheptyl bedeuten; oder $R^{10}$ und $R^{11}$ zusammen mit dem diese tragenden N-Atom einen 5- bis 7-gliedrigen heterocyclischen Ring bilden, der als Ringatome 2-6 Kohlenstoffatome und gegebenenfalls 1 oder 2 weitere gleiche oder verschiedene Heteroatome aus der Reihe Sauerstoff, Schwefel und Stickstoff enthält und der gegebenenfalls durch einen Rest aus der Reihe $(C_1-C_4)$-Alkyl, Phenyl, Phenyl-$(C_1-C_4)$-alkyl, Benzoyl, 2- oder 3-Furoyl, 2-, 3-oder 4-Pyridyl und 2-, 4- oder 5-Pyrimidinyl substituiert ist, worin Phenyl, Phenylalkyl und Benzoyl ihrerseits jeweils im Phenylrest durch einen oder zwei gleiche oder verschiedene Reste aus der Reihe Methyl, Methoxy, Fluor, Chlor, Brom, Cyano, Nitro und Hydroxy, drei Methoxy oder ein Methylendioxy substituiert sein können;

X Sauerstoff bedeutet;

m = 0, 1, 2, 3, 4, oder 5 ist;

n = 0 oder 1 ist.

Besonders bevorzugt sind Verbindungen der Formel I, worin

$R^1$ Wasserstoff; Methyl; Ethyl; Propyl; Isopropyl; Phenyl; o-, m- oder p-Tolyl; o-, m- oder p-Chlorphenyl; o-, m-oder p-Fluorphenyl; o-, m- oder p-Methoxyphenyl oder 2,3-, 2,4-, 2,5-, 2,6-, 3,4- oder 3,5-Dimethoxyphenyl bedeutet;

$R^2$ Wasserstoff; Phenyl; o-, m- oder p-Tolyl; o-, m- oder p-Chlorphenyl; o-, m- oder p-Fluorphenyl; o-, m- oder p-Methoxyphenyl; 2,3-, 2,4-, 2,5-, 2,6-, 3,4 oder 3,5-Dimethoxyphenyl; Phenoxy; o-, m- oder p-Tolyloxy; o-, m- oder p-Chlorphenoxy; o-, m- oder p-Fluorphenoxy; o-, m- oder p-Methoxyphenoxy; 2,3-, 2,4-, 2,5-, 2,6-, 3,4- oder 3,5-Dimethoxyphenoxy; 2-, 3- oder 4-Benzylphenoxy; 2-, 3- oder 4-Phenethyloxy; 2-, 3- oder 4-Styryloxy; Benzoyl; o-, m- oder p-Toluoyl; o-, m- oder p-Chlorbenzoyl; o-, m- oder p-Fluorbenzoyl; o-, m- oder p-Methoxybenzoyl; 2,3-, 2,4-, 2,5-, 2,6-, 3,4- oder 3,5-Dimethoxybenzoyl; Hydroxy oder Methoxy bedeutet;

$R^1$ und $R^2$ zusammen für Benzyliden; o-, m- oder p-Methylbenzyliden; o-, m- oder p-Methoxybenzyliden,

2,3-, 2,4-, 2,5-, 2,6-, 3,4- oder 3,5-Dimethoxybenzyliden oder 2-Phenyl-1-cyclopropyl stehen;
$R^6$ Wasserstoff oder Methyl bedeutet;
$R^7$ Wasserstoff; Methyl; Cyano; Benzoyl; o-, m- oder p-Methoxybenzoyl; o-, m- oder p-Chlorbenzoyl; 2,3-, 2,4-, 2,5-, 2,6-, 3,4- oder 3,5-Dimethoxybenzoyl; Acetyl; Methoxycarbonyl oder Ethoxycarbonyl bedeutet;
$R^8$ Cyano; Formyl; Benzoyl; o-, m- oder p-Methoxybenzoyl; o-, m- oder p-Chlorbenzoyl; 2,3-, 2,4-, 2,5-, 2,6-, 3,4-oder 3,5-Dimethoxybenzoyl; Acetyl; Methoxycarbonyl; Ethoxycarbonyl oder einen Rest der Formel

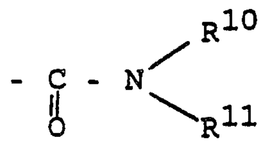

bedeutet;
$R^9$ Methyl; Ethyl; Benzyl oder Benzhydryl bedeutet;
$R^{10}$ und $R^{11}$ gleich oder verschieden sind und Wasserstoff; ($C_1$-$C_6$)-Alkyl; Phenyl oder Phenyl-($C_1$-$C_4$)-alkyl, worin der Phenylrest jeweils durch einen oder zwei gleiche oder verschiedene Reste aus der Reihe Methyl, Methoxy, Fluor und Chlor, drei Methoxy, oder ein Methylendioxy substituiert sein kann; Cyclopentyl oder Cyclohexyl bedeutet; oder
$R^{10}$ und $R^{11}$ zusammen mit dem diese tragenden N-Atom einen 5-bis 7-gliedrigen heterocyclischen Ring bilden, der als Ringatome 2-6 Kohlenstoffatome und gegebenenfalls 1 oder 2 weitere gleiche oder verschiedene Heteroatome aus der Reihe Sauerstoff, Schwefel und Stickstoff enthält und der gegebenenfalls durch Methyl, Phenyl, Phenyl-($C_1$-$C_4$)-alkyl, Benzoyl, Pyridyl oder Pyrimidinyl monosubstituiert ist, worin Phenyl, Phenylalkyl und Benzoyl ihrerseits jeweils im Phenylrest durch ein oder zwei gleiche oder verschiedene Reste aus der Reihe Methyl, Methoxy, Fluor, Chlor, Cyano and Nitro, oder drei Methoxy substituiert sein können;
X Sauerstoff bedeutet;
m = 0, 1, 2, 3, 4, oder 5 ist und
n = 0 oder 1 ist.

Insbesondere bevorzugt sind solche Verbindungen der Formel I, in welcher
$R^2$ Wasserstoff bedeutet;
$R^6$ Wasserstoff bedeutet;
$R^8$ Wasserstoff bedeutet;
X Sauerstoff bedeutet;
m = 1, 2, 3 oder 4 ist und
n = 0 oder 1 ist.

Die Erfindung betrifft auch ein Verfahren zur Herstellung von Verbindungen der Formel I, das dadurch gekennzeichnet ist, daß man

a) eine Verbindung der Formel II

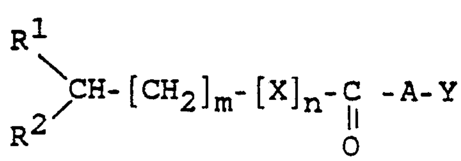

(II)

in welcher $R^1$, $R^2$, A, X, m und n die gleiche Bedeutung wie in Formel I haben und Y Hydroxy, ($C_1$-$C_{10}$)-Alkoxy, ($C_6$-$C_{12}$)-Aryloxy, ($C_6$-$C_{12}$)-Aryl-($C_1$-$C_6$)-alkoxy oder den Rest eines aktivierten Säurederivats bedeuten, mit einer Verbindung der Formel III umsetzt,

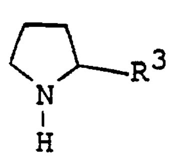

(III)

in welcher $R^3$ die gleiche Bedeutung wie in Formel I hat; oder

b) für den Fall daß $R^3$ Hydroxymethyl oder Formyl bedeutet, eine Verbindung der Formel II mit der Verbindung der Formel IV umsetzt,

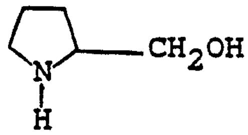

(IV)

und gegebenenfalls anschließend die Alkoholfunktion zum Aldehyd oxidiert; oder

$c_1$) für den Fall, daß $R^3$ den Rest

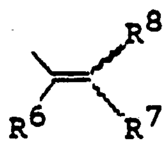

bedeutet, eine Verbindung der Formel V,

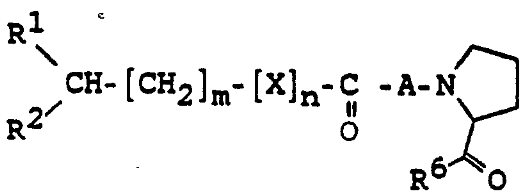

(V)

in welcher $R^1$, $R^2$, $R^6$, A, X, m und n die gleiche Bedeutung wie in Formel I haben, in Gegenwart einer Base umsetzt mit einer Verbindung der Formel VI

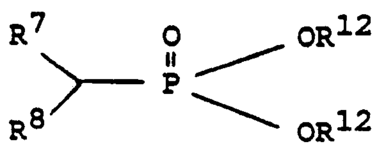

(VI)

in welcher $R^7$ und $R^8$ die gleiche Bedeutung wie in Formel I haben und $R^{12}$ für $(C_1-C_4)$-Alkyl, Phenyl oder Phenyl-$(C_1-C_4)$-alkyl steht; oder

$c_2$) eine Verbindung der unter ($c_1$) definierten Formel V umsetzt mit einer Verbindung der Formel VII

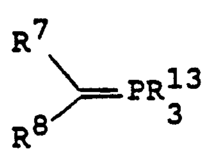

(VII)

in welcher $R^7$ und $R^8$ die gleiche Bedeutung wie in Formel I haben und $R^{13}$ für Phenyl, $(C_1-C_6)$-Alkylphenyl oder Di-$(C_1-C_6)$-alkylphenyl steht; oder

$c_3$) zur Herstellung einer Verbindung der Formel I, worin $R^8$ für einen Rest der Formel

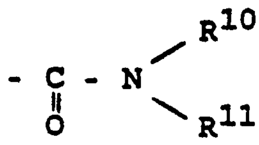

steht,

eine Verbindung der Formel I, in welcher $R^8$ ($C_1$-$C_6$)-Alkoxycarbonyl bedeutet und die übrigen Reste und Variablen wie oben definiert sind, umsetzt mit einer Verbindung der Formel VIII

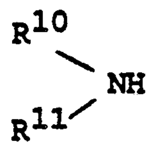

(VIII)

in welcher $R^{10}$ und $R^{11}$ die gleiche Bedeutung wie in Formel I haben; oder

$c_4$) eine Verbindung der Formel I, in welcher $R^8$ Carboxy bedeutet und die übrigen Reste und Variablen wie oben definiert sind, nach deren Umwandlung in ein aktiviertes Derivat mit einer Verbindung der unter ($c_3$) definierten Formel VIII umsetzt; oder

d) für den Fall, daß $R^3$ den Rest $-CO-CF_3$, $-CO-CF_2-R^{10}$ oder $-CO-CF_2-CO_2-R^9$ bedeutet, eine Verbindung der Formel I, in welcher $R^3$ Formyl bedeutet, mit einer Verbindung der Formel IX umsetzt,

$Z-CF_2-R^{14}$ (IX)

in welcher $R^{14}$ die Bedeutung von $R^{10}$ hat oder für Fluor oder $-CO_2R^9$ steht und Z Halogen, insbesondere Brom oder Jod bedeutet, und das erhaltene Produkt oxidert; oder

$d_1$) für den Fall, daß $R^3$ den Rest $-CO-CF_2-R^{10}$ darstellt, eine Verbindung der Formel I, in welcher $R^3$ für $-CO-CF_2-CO_2R^9$ steht, wobei $R^9$ Wasserstoff bedeutet, zunächst mit einer Verbindung der Formel X umsetzt,

$R^{15}-N(H)-O-R^{16}$ (X)

in welcher $R^{15}$ und $R^{16}$ gleich oder verschieden und unabhängig voneinander ($C_1$-$C_4$)-Alkyl bedeuten können, oder mit den sie tragenden Heteroatomen einen 5-7-gliedrigen Ring bilden, das Produkt anschließend mit einer Verbindung der Formel XI umsetzt,

$R^{10}-Mg-W$ (XI)

in welcher $R^{10}$ die gleiche Bedeutung wie in Formel I hat, und W für Halogen, insbesondere für Chlor, Brom oder Jod steht, und das erhaltene Produkt oxidiert; oder

$d_2$) für den Fall, daß $R^3$ für

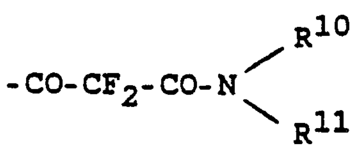

steht, eine Verbindung der Formel XII

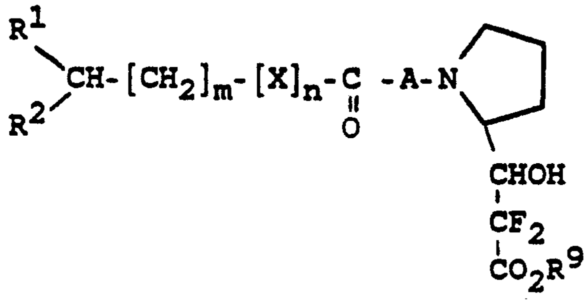

(XII)

in welcher $R^1$, $R^2$, $R^9$, A, X, m und n die gleiche Bedeutung wie in Formel I haben, mit einer Verbindung der Formel VIII umsetzt und anschließend oxidiert; oder

e) für den Fall, daß $R^3$ für $-CO-CO_2R^9$ steht, eine Verbindung der Formel XIII,

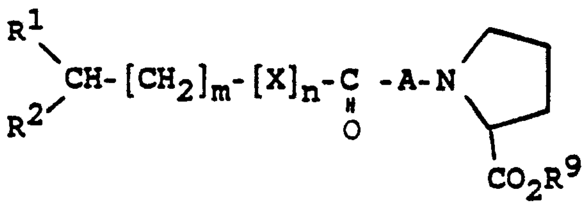

(XIII)

in welcher $R_1$, $R^2$, $R^9$, A, X, m und n die gleiche Bedeutung wie in Formel I haben, zunächst mit der Verbindung der Formel XIV

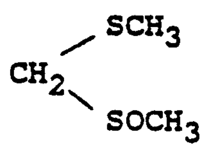

(XIV)

in Gegenwart einer Base umsetzt, und das Produkt anschließend mit einem Kupfer- oder Quecksilbersalz in einem Alkohol $R^9$-OH als Lösungsmittel zur Reaktion bringt; oder

f) für den Fall, daß $R^3$ den Rest

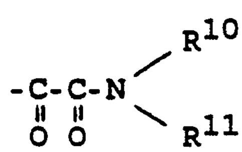

bedeutet, eine Verbindung der Formel I, in welcher $R^3$ $-CO-CO_2R^9$ bedeutet, zunächst gegebenenfalls zur freien Säure verseift und anschließend mit einer Verbindung der Formel VIII umsetzt und die nach (a)-(f) erhaltenen Verbindungen gegebenenfalls in ihre physiologisch verträglichen Salze überführt.

Bei Verfahrensvariante a) verfährt man, falls Y Hydroxy bedeutet, vorzugsweise in Analogie zu den in der Peptidchemie gebräuchlichen Amidknüpfungsverfahren, wie sie beispielsweise in Houben-Weyl, Band 15/2, Seite 1-364, in Bodanszky, "Principles bzw. Practice in Peptide Synthesis", Berlin, 1984 und den US-Patenten 4 331 592 und 4 426 325 beschrieben werden, indem man die Umsetzung in einem organischen Lösungsmittel wie DMF, $CH_2Cl_2$, DMA in Gegenwart von Kupplungshilfsmitteln, wie Carbodiimiden (z.B. Dicyclohexylcarbodiimid), Diphenylphosphorylazid, Alkanphosphorsäureanhydriden, Dialkylphosphinsäureanhydriden oder N,N-Succinimidylcarbonaten in einem Lösungsmittel wie $CH_3CN$ durchgeführt. Die Verbindungen der Formel II, in welcher Y Hydroxy bedeutet, können in Aktivester (z.B. mit 1-Hydroxybenzotriazol), gemischte Anhydride (z.B. mit Chlorameisensäureestern), Azide oder Carbodiimid-Derivate überführt und damit aktiviert werden (vgl. Schröder, Lübke, The Peptides, Band 1, New York 1965, Seiten 76-136). Die Reaktion wird vorzugweise zwischen -20 °C und dem Siedepunkt des Reaktionsgemisches durchgeführt.

Falls Y ($C_1$-$C_{20}$)-Alkoxy, ($C_6$-$C_{12}$)-Aryloxy oder ($C_6$-$C_{12}$)-Aryl-($C_1$-$C_6$)-alkoxy bedeutet, kann man die Umsetzung in einem geeigneten organischen Lösungsmittel wie einem niederen Alkohol, Dimethylformamid oder Dimethylsulfoxid, vorzugsweise in Ethanol, bei 20 °C bis zum Siedepunkt des Reaktionsgemisches vorzugsweise bei 40-80 °C durchführen.

Bei Verfahrensvariante (b) führt man die Verknüpfung der Verbindung der Formel II mit der Verbindung der Formel IV analog der in Variante (a) beschriebenen Verfahrensweise durch. Für die anschließende Oxidation kommen folgende Oxidationsmittel in Betracht:

Mangandioxid, Natrium- oder Kaliumdichromat; Jones Reagenz ($CrO_3$ in wäßriger Schwefelsäure), N-Bromacetamid, N-Bromsuccinimid, Dimethylsulfoxid, Cerammonnitrat, $CrO_3$ in Pyridin, tert.-Butylchromat, Dipyridin-$CrO_3$, Kaliumhypochlorit, Jodosobenzol. Als Reaktionsmedium sind Petrolether, Benzol, Tetrachlorkohlenstoff oder, im Falle von $MnO_2$, verdünnte Schwefelsäure geeignet. Die Oxidation wird zwischen 0 °C und dem Siedepunkt des Reaktionsgemisches durchgeführt. Bevorzugt ist die Oxidation mit Dimethylsulfoxid mit verschiedenen Zusätzen, wie sie beispielsweise in Houben-Weyl, Band E 3, Seiten 275-281 beschrieben wird. Insbesondere bevorzugt sind die Dimethylsulfoxid-Oxidation in Gegenwart von Oxalylchlorid sowie das in J. Org. Chem. 48 [1983] 4155 beschriebene Verfahren.

Die Umsetzung gemäß Verfahrensvariante ($c_1$) (vgl. hierzu Chem. Revs. 74 [1974] 87) führt man in einem geeigneten organischen Lösungsmittel wie einem Ether (z.B. Diethylether, THF, Glyme, Diglyme), Kohlenwasserstoff (z.B. Cyclohexan, Toluol, Xylol), oder Amid (z.B. DMF, DMA) in Gegenwart einer Base wie einem Alkalialkoholat, Alkalihydrid, Alkalihydroxid, Alkaliamid, 1,5-Diazabicyclo-(4,3,0)-5-nonen, 1,8-Diazabicyclo(5,4,0)-7-undecen oder eine Lithiumdialkylamid bei -80 °C bis +50 °C, vorzugsweise bei -40 °C bis +30 °C durch.

Verfährt man nach Verfahrensvariante ($c_2$) (vgl. hierzu Accts. Chem. Res. 7 [1974] 6 und 85), so verwendet man als Reaktionsmedium ein aprotisches organisches Lösungsmittel wie einen Kohlenwasserstoff (z.B. Benzol, Toluol, Xylol, Tetrahydronaphthalin), einen chlorierten Kohlenwasserstoff (z.B. Dichlormethan, Dichlorethan), ein Amid (wie DMF, DMA) oder DMSO bei einer Reaktionstemperatur von 0 °C bis zum Siedepunkt des Reaktionsgemisches, vorzugsweise von 40 °C bis zum Siedepunkt des Reaktionsgemisches.

Die Umamidierung gemäß Verfahrensvariante ($c_3$) wird in einem polaren protischen oder aprotischen Lösungsmittel wie einem niederen Alkohol (z.B. Ethanol, n-Butanol), DMSO oder DMF bei 0 °C bis zum Siedepunkt des Rekationsgemisches, vorzugsweise bei 30 °C bis zum Siedepunkt des Reaktionsgemisches oder ohne Lösungsmittel bei 0 bis 100 °C durchgeführt.

Bei Verfahrensvariante ($c_4$) wird eine Verbindung der Formel I ($R^8$ = COOH) in ihr aktiviertes Derivat wie Säurechlorid, Anhydrid oder Aktivester (vgl. Schröder, Lübke "The Peptides", Volume I, New York, 1965, Seiten 77-128) überführt und in einem geeigneten organischen Lösungsmittel vorzugsweise aus der Reihe der bei Variante ($c_3$) genannten Lösungsmittel bei einer Temperatur zwischen -20 °C und dem Siedepunkt des Reaktionsgemisches vorzugsweise zwischen 0 °C und dem Siedepunkt des Reaktionsgemisches weiter umgesetzt.

Bei Verfahrensvariante (d) führt man die Umsetzung der Verbindungen der Formeln I und IX, vorzugsweise in einem inerten Lösungsmittel wie einem Ether, Dimethylformamid unter Zuhilfenahme eines Metalls wie Lithium, Natrium, Kalium, Magnesium oder Zink, wobei das letztere bevorzugt ist, bei 0 °C bis zum Siedepunkt des Lösungsmittels, vorzugsweise bei 20 bis 80 °C mit oder ohne zusätzliche Behandlung mit Ultraschall durch. Die anschließende Oxidation erfolgt unter den in Verfahrensvariante (b) beschriebene Bedingungen.

Bei Verfahrensvariante ($d_1$) führt man die Kupplung der Verbindungen (I) und (X) unter den bei Verfahrensvariante (a) beschriebenen Peptidknüpfungsmethoden durch. Die anschließende Reaktion mit der Verbindung der Formel XI erfolgt in einem inerten organischen Lösungsmittel, insbesondere einem Ether wie Diethylether oder Tetrahydrofuran bei 0 °C bis zum Siedepunkt des Lösungsmittels; das erhaltene Produkt wird mit den in Verfahrensvariante (b) beschriebenen Oxidationsmittel oxidiert.

Bei Verfahrensvariante ($d_2$) verfährt man, wie für die Variante (a) beschrieben und führt die anschließende Oxidation, wie in Variante (b) beschrieben, durch.

Bei Verfahrensvariante (e) führt man die Umsetzung der Verbindung der Formel XIII mit der Verbindung der Formel XIV in einem inerten organischen Lösungsmittel, vorzugsweise einem Ether wie Diethylether, Tetrahydrofuran, Dioxan oder 1,2-Dimethoxyethan in Gegenwart einer starken Base, vorzugsweise einem Alkalihydrid, einem Alkalialkoholat, einem Alkaliamid oder einer Alkylalkali-Verbindung, bei 0 °C bis zum

Siedepunkt des Lösungsmittels durch. Die anschließende Umsetzung mit einem Kupfer- oder Quecksilbersalz erfolgt in einem Alkohol $R^9OH$, in reiner Form oder in Mischung mit Wasser, bei 0 °C bis zum Siedepunkt des Lösungsmittels.

Bei Verfahrensvariante (f) erfolgt die Hydrolyse unter sauren oder basischen Bedingungen in Wasser oder einer Mischung aus Wasser und einem niederen Alkohol bei 0 °C bis 100 °C. Die anschließende Kupplung mit der Verbindung der Formel VIII erfolgt unter den in Verfahrensvariante (a) beschriebenen Amidbildungsverfahren.

Verbindungen der Formel II erhält man in Analogie zu bekannten Verfahren, beispielsweise den in den europäischen Patentanmeldungen EP-A 172458 und EP-A 207742 beschriebenen Verfahren.

Insbesondere gelingt ihre Herstellung durch Verknüpfung einer Verbindung der Formel XV

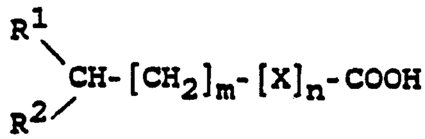

(XV)

mit einer Verbindung der Formel XVI

H-A-Y (XVI)

unter den in Verfahrensvariante (a) beschriebenen Peptidbildungsmethoden.

Verbindungen der Formel V mit $R^6 \neq H$ erhält man aus Verbindungen der Formel I mit $R^3$ = Formyl durch Umsetzung mit einer Verbindung der Formel XVII

$R^6$-Mg-W (XVII)

unter den in Verfahrensvariante ($d_1$) beschriebenen Bedingungen und anschließend Oxidation unter den in Verfahrensvariante (b) beschriebenen Bedingungen. Verbindungen der Formel XIII erhält man aus Verbindungen der Formel II und Verbindungen der Formel XVIII

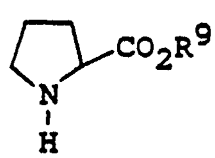

(XVIII)

unter den in Verfahrensvariante (a) beschriebenen Amidbildungsbedingungen.

Die erfindungsgemäßen Verbindungen der Formel I sind Hemmstoffe der Prolyl-Endopeptidase (EC 3.4.21.26). Von diesem Enzym ist bekannt, daß es Neuropeptide wie Substanz P, Neurotensin, LHRH, TRH, Vasopressin sowie Angiotensin II abbaut (Life Sci. 33, 2149 (1983)). Diese Neuropeptide sind mit wichtigen Funktionen im Zentralnervensystem assoziiert. Durch Hemmung ihres Abbaus mittels Hemmung der Prolyl-Endopeptidase werden durch Verbindungen der Formel I verschiedenartige Wirkungen im ZNS ausgelöst, insbesondere antiamnestische, antipsychotische, anxiolytische und antidepressive Wirkungen.

Verbindungen der Formel I eignen sich daher zur Behandlung verschiedener Erkrankungen des Zentralnervensystems, insbesondere als Nootropika und Antipsychotika bei Warmblütern, vorzugsweise beim Menschen. Die Anwendung der erfindungsgemäßen Verbindungen kann intravenös, subkutan oder peroral für sich allein oder in Kombination mit anderen ZNS-wirksamen Substanzen erfolgen.

Die Dosierung liegt je nach Art und Schwere der zu behandelnden Erkrankung bei 0,0001-100 mg/kg/Tag, insbesondere bei 0,001-10 mg/kg/Tag. Sie kann in schweren Fällen auch erhöht werden, da toxische Eigenschaften bisher nicht beobachtet wurden.

Die erfindungsgemäßen Verbindungen können oral oder parenteral in entsprechender pharmazeutischer Zubereitung verabreicht werden. Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesiumcarbonat, Kaliumphosphat, Milchzucker, Glucose oder Stärke, insbesondere Maisstärke verwendet werden. Dabei kann die Zubereitung sowohl als Trocken- oder Feuchtgranulat erfolgen. Als ölige Trägerstoffe oder Lösungsmittel kommen beispielsweise pflanzliche und tierische Öle in Betracht, wie Sonnenblumenöl oder Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren, oder weitere Hilfsstoffe in Lösung, Suspension oder Emulsion gebracht. Als Lösungsmittel für die neuen aktiven Verbindungen und die entsprechenden physiologisch verträglichen Salze kommen z.B. in Frage: Wasser, physiologische Kochsalzlösungen oder Alkohole, z.B. Ethanol, Propandiol, oder Glycerin, daneben auch Zuckerlösungen wie Glucose- oder Mannitlösungen, oder auch eine Mischung aus den verschiedenen genannten Lösungsmitteln.

Die folgenden Beispiele sollen die Erfindung erläutern, ohne sie auf die genannten Verbindungen zu beschränken.

**Beispiel 1**

N-[N-(4-Phenyl-butyryl)-(2S,3aS,6aS)-octahydrocyclopenta[b]pyrrol-2-carbonyl]-S-prolinol

a) N-(4-Phenyl-butyryl)-(2S,3aS,6aS)-octahydrocyclopenta[b]pyrrol-2-carbonsäure-benzylester

4,92 g (30 mmol) 4-Phenylbuttersäure werden zusammen mit 8,4 ml Triethylamin in 200 ml trockenem Tetrahydrofuran gelöst. Unter Eiskühlung tropft man 2,9 ml Chlorameisensäureethylester zu. Nach 1 Stunde Rühren bei 0°C werden 7,4 g (2S,3aS,6aS)-Octahydrocyclopenta[b]pyrrol-2-carbonsäure-benzylester-hydrochlorid zugesetzt und weitere 12 Stunden bei Raumtemperatur gerührt. Nach Filtration wird eingeengt und der Rückstand in 3 % $NaHCO_3$/Ethylacetat aufgenommen. Die organische Phase wird abgetrennt und je 1 mal mit 10 %iger Citronensäure, 3 %iger $NaHCO_3$-Lösung und gesättigter Kochsalzlösung gewaschen, mit $mgSO_4$ getrocknet und eingeengt. Man erhält nach Chromatographie an Kieselgel mit Ethylacetat/Cyclohexan (1:2) als Laufmittel 9,05 g der Titelverbindung als farbloses Öl.

$^1$H-NMR ($CDCl_3$) $\delta$ = 7,4 (s, 5H); 7,3 (s, 5H); 5,2 (s, 2H); 4,8-4,5 (dd, 1H); 4,5-3,9 (m, 1H); 2,8-1,4 (m, 15H)ppm.

b) N-[N-(4-Phenylbutyryl)-(2S,3aS,6aS)-octahydrocyclopenta[b]-pyrrol-2-carbonyl]-S-prolinol

9,05 (23,1 mmol) der Verbindung aus Beispiel 1a, werden in 200 ml Ethanol mit 100 mg Pd/C (10 %) bei Normaldruck und Raumtemperatur hydriert. Nach Ende der Wasserstoff-Aufnahme wird der Katalysator abgesaugt und die Lösung eingeengt. Das Rohprodukt (7 g) wird zusammen mit 2,3 ml S-Prolinol, 3,5 g 1-Hydroxybenzotriazol und 3 ml N-Ethylmorpholin in 150 ml Dimethylformamid gelöst. Bei 0°C gibt man 4,75 g Dicyclohexylcarbodiimid zu und rührt 16 Stunden bei Raumtemperatur. Nach Absaugen des Niederschlags und Waschen mit Ethylacetat wird mit Ethylacetat verdünnt und je 1 mal mit 3 % $NaHCO_3$, 10 %iger Citronensäure, 3 % $NaHCO_3$, Wasser und gesättigter Kochsalzlösung gewaschen. Nach Trocknen mit $MgSO_4$ wird eingeengt. Man erhält 8 g der Titelverbindung als blaßgelbes Öl.

$^1$H-NMR ($CDCl_3$) $\delta$ = 7,35-7,15 (m, 5H); 5,15 (t, 1H); 4,65 (t, 1H); 4,35 (m, 1H); 4,2-4,0 (m, 2H); 4,0-3,35 (m, 5H); 2,8-2,6 (m, 3H); 2,4-2,2 (m, 3H); 2,1-1,5 (m, 12H)ppm.

**Beispiel 2**

N-[N-(4-Phenylbutyryl)-(2S,3aS,6aS)-octahydrocyclopenta-[b]pyrrol-2-carbonyl]-S-prolinal

2,1 ml Oxalylchlorid werden in 100 ml Dichlormethan auf - 78°C gekühlt. 3,2 ml Dimethylsulfoxid werden zugetropft. Nach 5 Minuten gibt man 8 g (20,8 mmol) der Verbindung aus Beispiel 1b in 50 ml Dichlormethan langsam zu. Nach weiteren 15 Minuten werden 14,4 ml Triethylamin zugesetzt und die Mischung auf Raumtemperatur aufgewärmt. Nach Verdünnen mit Wasser wird die organische Phase abgetrennt, mit $MgSO_4$ getrocknet und eingeengt. Das Rohprodukt wird an 500 g Kieselgel mit Essigester als Laufmittel chromatographiert. Man erhält 6,2 g der Titelverbindung als farbloses Öl.

$^1$H-NMR ($CDCl_3$) $\delta$ = 9,5 (d, 1H); 7,3-7,1 (m, 5H); 4,7 (dd, 1H); 4,6 (m, 1H); 4,15 (m, 1H); 3,9 (m, 1H); 3,55 (m, 1H); 2,8 (m, 1H); 2,7 (t, 2H); 2,45-2,2 (m, 3H); 2,1-1,4 (m, 12H)ppm.

**Beispiel 3**

3-[N-[N-(4-Phenylbutyryl)-(2S,3aS,6aS)-octahydrocyclopenta-[b]pyrrol-2-carbonyl]-2S-pyrrolidinyl]-3-hydroxy-2,2-difluor-propionsäuremethylester

1,3 g Zinkpulver werden mit 3,8 g (20 mmol) Bromdifluoressigsäuremethylester in 30 ml THF 1 Minute zum Sieden erhitzt. 6,15 g (16,8 mmol) der Verbindung aus Beispiel 2 werden zugegeben und noch 15 Minuten zum Sieden erhitzt. Nach Abkühlen wird mit Essigester/Wasser verdünnt, der Niederschlag abgesaugt, die Phasen getrennt und die organische Phase mit gesättigter Kochsalzlösung gewaschen. Nach Trocknen mit $MgSO_4$ wird eingeengt und an Kieselgel mit Ethylacetat/Cyclohexan (4:1) als Laufmittel chromatographiert. Man erhält 2,9 g der Titelverbindung als Öl.

$^1$H-NMR ($CDCl_3$) $\delta$ = 7,3-7,1 (m, 5H); 5,75 (d); 5,0 (t); 4,75-4,6 (m, 2H); 4,3-3,6 (m, 5H); 3,9 (s, 3H); 3,5 (m, 1H); 2,8 (m, 1H); 2,65 (2t, 2H); 2,4-2,2 (m, 3H); 2,1-1,5 (m, 12H)ppm.

**Beispiel 4**

3-[N-[N-(4-Phenylbutyryl)-(2S,3aS,6aS)-octahydrocyclopenta-[b]pyrrol-2-carbonyl]-2S-pyrrolidinyl]-3-hydroxy-2,2-difluor-propionsäure-N-benzylamid

0,5 g (1 mmol) der Verbindung aus Beispiel 3 werden mit 0,54 g (5 mmol) Benzylamin in 15 ml Ethanol 3 Stunden am Rückfluß gekocht. Nach Einengen wird in Essigester aufgenommen und mit 1 N HCl und gesättigter NaCl-Lösung gewaschen, über $MgSO_4$ getrocknet und eingeengt. Man erhält 0,6 g der Titelverbindung als Öl.

$^1$H-NMR ($CDCl_3$) $\delta$ = 7,35-7,1 (m, 10H); 4,7-4,4 (m, 3H); 4,2-3,8 (m, 2H); 3,5 (m, 1H); 2,8-2,6 (m, 3H); 2,45-2,2 (m, 3H); 2,2-1,5 (m, 12H)ppm.

**Beispiel 5**

3-[N-[N-(4-Phenylbutyryl)-(2S,3aS,6aS)-octahydrocyclopenta-[b]pyrrol-2-carbonyl]-2S-pyrrolidinyl]-3-hydroxy-2,2-difluor-propionsäure-N-(2-phenyl-ethyl)-amid

Durch Umsetzung von 0,5 g (1 mmol) der Verbindung aus Beispiel 3 mit 0,6 g (5 mmol) 2-Phenylethylamin analog der in Beispiel 4 angegebenen Vorschrift erhält man 0,8 g der Titelverbindung als Öl.

$^1$H-NMR ($CDCl_3$) $\delta$ = 7,4-7,1 (m, 10H); 4,7-4,5 (m, 2H); 4,2-3,3 (m, 5H); 2,85 (t, 2H); 2,8 (m, 1H); 2,65 (t,2H); 2,4-2,2 (m, 3H); 2,1-1,5 (m, 12H)ppm.

**Beispiel 6**

3-[N-[N-(4-Phenylbutyryl)-(2S,3aS,6aS)-octahydrocyclopenta[b]-pyrrol-2-carbonyl]-2S-pyrrolidinyl]-3-hydroxy-2,2-difluor-propionsäure-N-(2-picolyl)-amid

Durch Umsetzung von 0,5 g (1 mmol) der Verbindung aus Beispiel 3 mit 0,55 g (5 mmol) 2-Picolylamin analog dem in Beispiel 4 angegebenen Verfahren erhält man 0,5 g der Titelverbindung als Öl.

$^1$H-NMR ($CDCl_3$) $\delta$ = 8,55 (m, 1H); 7,8-7,6 (m, 2H); 7,35-7,1 (m, 6H); 4,8-4,5 (m, 2H); 4,2-3,7 (m, 3H); 3,5 (m, 2H); 2,8 (m, 1H); 2,65 (m, 3H); 2,3 (m, 3H); 2,2-1,5 (m, 12H)ppm.

**Beispiel 7**

3-[N-[N-(4-Phenylbutyryl)-(2S,3aS,6aS)-octahydrocyclopenta[b]-pyrrol-2-carbonyl]-2S-pyrrolidinyl]-3-hydroxy-2,2-difluor-propionsäure-N-(1R-phenyl-ethyl)-amid

Durch Umsetzung von 0,5 g (1 mmol) der Verbindung aus Beispiel 3 mit 0,6 g (5 mmol) 1R-Phenylethylamin analog dem in Beispiel 4 angegebenen Verfahren erhält man 0,64 g der Titelvebrindung als Öl.

$^1$H-NMR ($CDCl_3$) $\delta$ = 7,5-7,1 (m, 10H); 5,2-4,6 (m, 2H); 4,4-3,8 (m, 4H); 3,5 (m, 1H); 2,8-2,5 (m, 3H); 2,4-2,2 (m, 3H); 2,1-1,5 (m, 12H); 1,7+1,6 (2d, 3H)ppm.

**Beispiel 8**

3-[N-[N-(4-Phenylbutyryl)-(2S,3aS,6aS)-octahydrocyclopenta[b]-pyrrol-2-carbonyl]-2S-pyrrolidinyl]-2,2-difluor-3-oxo-propionsäuremethylester

Analog dem in Beispiel 2 angegebenen Verfahren erhält man aus 0,9 g der Verbindung aus Beispiel 3 durch Umsetzung mit 0,16 ml Oxalylchlorid, 0,3 ml DMSO und 1,2 ml Triethylamin nach Chromatographie an Kieselgel mit Ethylacetat/Cyclohexan (2:1) als Laufmittel 0,5 g der Titelverbindung als farbloses Öl.

$^1$H-NMR ($CDCl_3$) $\delta$ = 7,3-7,1 (m, 5H); 5,0 (dd, 1H); 4,7 (m, 1H); 4,15 (m, 1H); 3,9 (s, 3H); 3,95-3,85 (m, 1H); 3,6-3,4 (m, 1H); 2,8-2,6 (m, 3H); 2,4-2,2 (m, 3H); 2,2-1,5 (m, 12H)ppm.

**Beispiel 9**

3-[N-[N-(4-Phenylbutyryl)-(2S,3aS,6aS)-octahydrocyclopenta[b]-pyrrol-2-carbonyl]-2S-pyrrolidinyl]-2,2-difluor-3-oxo-propionsäure-N-benzylamid

Analog dem in Beispiel 2 angegebenen Verfahren erhält man aus 0,6 g der Verbindung aus Beispiel 4 durch Umsetzung mit 0,1 ml Oxalylchlorid, 0,16 ml DMSO und 0,7 ml Triethylamin nach Chromatographie an Kieselgel mit Ethylacetat/Cyclohexan (4:1) als Laufmittel 0,4 g der Titelverbindung als Öl.

$^1$H-NMR ($CDCl_3$) $\delta$ = 8,0 (s, 1H); 7,4-7,1 (m, 10H); 5,08 (dd, 1H); 4,7-4,4 (m, 3H); 4,1 (m, 1H); 3,9 (m, 1H); 3,6 (m, 1H); 2,8-2,6 (m, 3H); 2,4-2,2 (m, 3H); 2,2-1,4 (m, 12H)ppm.

**Beispiel 10**

30[N-[N-(4-Phenylbutyryl)-(2S,3aS,6aS)-octahydrocyclopenta[b]-pyrrol-2-carbonyl]-2S-pyrrolidinyl]-2,2-difluor-3-oxo-propionsäure-N-(2-phenylethyl)-amid

Analog dem in Beispiel 2 angegebenen Verfahren erhält man aus 0,8 g der Verbindung aus Beispiel 5 durch Umsetzung mit 0,14 mol Oxalylchlorid, 0,23 ml DMSO und 0,9 ml Triethylamin nach Chromatographie an Kieselgel mit Ethylacetat/Cyclohexan (4:1) als Laufmittel 0,33 g der Titelverbindung als Öl.

$^1$H-NMR ($CDCl_3$) $\delta$ = 7,8 (s, 1H); 7,3-7,1 (m, 10H); 5,03 (dd, 1H); 4,7 (dd, 1H); 4,15 (m, 1H); 3,95 (m, 1H); 3,55 (m, 3H); 2,83 (t, 2H), 2,8 (m, 1H); 2,65 (t, 2H); 2,45-2,2 (m, 3H); 2,2-1,5 (m, 12H)ppm.

**Beispiel 11**

3-[N-[N-(4-Phenylbutyryl)-(2S,3aS,6aS)-octahydrocyclopenta[b]-pyrrol-2-carbonyl]-2S-pyrrolidinyl]-2,2-difluor-3-oxo-propionsäure-N-(2-picolyl)-amid

Analog dem in Beispiel 2 angegebenen Verfahren erhält man aus 0,5 g der Verbindung aus Beispiel 6 durch Umsetzung mit 80 $\mu$l Oxalylchlorid, 0,14 ml DMSO und 0,6 ml Triethylamin nach Chromatographie an Kieselgel mit Ethylacetat/Cyclohexan (7:1) als Laufmittel 0,27 g der Titelverbindung als Öl.

$^1H$-NMR ($CDCl_3$) δ = 8,53 (m, 1H); 8,35 (s, 1H); 7,65 (m, 1H); 7,3-7,1 (m, 7H); 5,65 (dd, 1H); 4,65 (m, 3H); 4,1 (m, 1H); 3,9 (m, 1H); 3,6 (m, 1H); 2,75 (m, 1H); 2,65 (t, 2H); 2,35 (m, 3H); 2,2-1,4 (m, 12H)ppm.

**Beispiel 12**

3-[N-[N-(4-Phenylbutyryl)-(2S,3aS,6aS)-octahydrocyclopenta[b]-pyrrol-2-carbonyl]-2S-pyrrolidinyl]-2,2-difluor-3-oxo-propionsäure-N-(1R-phenylethyl)-amid

Analog dem in Beispiel 2 angegebenen Verfahren erhält man aus 0,5 g der Verbindung aus Beispiel 7 durch Umsetzung mit 0,1 ml Oxalylchlorid, 0,18 ml DMSO und 0,76 ml Triethylamin nach Chromatographie an Kieselgel mit Ethylacetat/Cyclohexan (4:1) als Laufmittel 0,12 g der Titelverbindung als Öl.

$^1H$-NMR ($CDCl_3$) δ = 8,1 (d, 1H); 7,3-7,1 (m, 10H); 5,1 (m, 2H); 4,7 (m, 1H); 4,15 (m, 1H); 3,95 (m, 1H); 3,6 (m, 1H); 2,8 (m, 1H); 2,7 (m, 2H); 2,5-2,3 (m, 5H); 2,2-1,5 (m, 12H); 1,55 (d, 3H)ppm.

**Beispiel 13**

N-(4-Phenylbutyryl)-1.2.3.4-tetrahydroisochinolin-3S-carbonsäurepyrrolidid

a) N-(4-Phenylbutyryl)-1.2.3.4-tetrahydroisochinolin-3S-carbonsäure tert. butylester

3,98 g (22 mmol) 4-Phenylbuttersäure werden zusammen mit 6,1 ml (44 mmol) Triethylamin in 200 ml trockenem THF gelöst. Bei 0 °C werden 2,3 ml Chlorameisensäureethylester zugetropft. Nach 2 Stunden gibt man 6 g 1.2.3.4-Tetrahydroisochinolin-3S-carbonsäure-tert.butylester zu und rührt noch 12 Stunden. Nach Filtration wird eingeengt, in 3 % $NaHCO_3$/Ethylacetat aufgenommen und je 1 mal mit 10 % Citronensäure, 3 % $NaHCO_3$ und gesättigter Kochsalzlösung gewaschen, mit $MgSO_4$ getrocknet und eingeengt.

Chromatographie an Kieselgel mit Ethylacetat/Cyclohexan (1:2) als Laufmittel ergibt 3,7 g der Titelverbindung als Öl.

$^1H$-NMR ($CDCl_3$) δ = 7,15 (s, 5H); 7,1 (s, 4H); 5,45 + 4,6 (2m, 3H); 3,1 (m, 2H); 2,9-1,9 (m, 6H); 1,2 (s, 9H)ppm.

b) N-(4-Phenylbutyryl)-1.2.3.4-tetrahydroisochinolin-3S-carbonsäure

6,1 g der Verbindung aus Beispiel 13a) werden in 30 ml Trifluoressigsäure 6 Stunden bei Raumtemperatur gerührt. Nach Einengen im Vakuum wird 3 mal mit Toluol aufgenommen und eingeengt. Man erhält 5,7 g der Titelverbindung als Öl.

$^1H$-NMR ($CDCl_3$) δ = 9,95 (s, 1H); 7,2 (s, 9H); 5,5+4,6 (2m, 1H); 4,55 (s, 2H); 3,2 (m, 2H); 3,0-2,0 (m, 6H)ppm.

c) N-(4-Phenylbutyryl)-1.2.3.4-tetrahydroisochinolin-3S-carbonsäure-pyrrolidid

0,87 g (2,7 mmol) der Verbindung aus Beispiel 13b) werden zusammen mit 0,3 ml Pyrrolidin, 0,44 ml N-Ethylmorpholin und 0,49 g 1-Hydroxybenzotriazol in 15 ml trockenen Dimethylformamid gelöst. Man gibt 0,65 g Dicyclohexylcarbodiimid zu und rührt 18 Stunden bei Raumtemperatur. Nach Filtration wird mit Ethylacetat verdünnt und je 1 mal mit 3 % $NaHCO_3$, 10 % Citronensäure, 3 % $NaHCO_3$ und gesättigter Kochsalzlösung gewaschen, über $MgSO_4$ getrocknet und eingeengt. Chromatographie an Kieselgel (Laufmittel: Dichlormethan/Methanol = 20:1) ergibt 0,37 g der Titelverbindung als Öl.

$^1H$-NMR ($CDCl_3$) δ = 7,2 (s, 9H); 5,5-4,7 (2m, 1H); 4,5 (s, 2H); 3,7-3,4 (m, 4H); 3,2 (m, 2H); 3,0-2,0 (m, 10H)ppm.

**Beispiel 14**

N-[N-(4-Phenylbutyryl)-1.2.3.4-tetrahydroisochinolin-3S-carbonyl]-S-prolinol

Analog dem in Beispiel 1b) angegebenen Verfahren erhält man aus 3,2 g der Verbindung aus Beispiel 13b) und 1 ml Prolinol sowie 1,6 g 1-Hydroxybenzotriazol, 1,4 ml N-Ethylmorpholin und 2,4 g Dicyclohexylcarbodiimid 3,9 g der Titelverbindung nach Chromatographie an Keiselgel (Laufmittel: Dichlormethan/Methanol = 40:1).

$^1$H-NMR ($CDCl_3$) $\delta$ = 7,3-7,1 (m, 9H); 5,2-4,8 (m, 1H); 4,7-4,5 (m, 1H); 4,3-3,9 (m, 2H); 3,8-3,3 (m, 3H); 3,2-2,9 (m, 2H); 2,7 (m, 2H); 2,45 (m, 2H); 2,1-1,6 (m, 8H)ppm.

**Beispiel 15**

N-[N-(4-Phenylbutyryl)-1.2.3.4-Tetrahydroisochinolin-3S-carbonyl]-S-prolinal

Analog dem in Beispiel 2 angegebenen Verfahren erhält man aus 2,46 g (6 mmol) der Verbindung aus Beispiel 14 durch Umsetzung mit 1 ml Oxalylchlorid, 1,67 ml DMSO und 6,7 ml Triethylamin 0,7 g der Titelverbindung als Öl.

$^1$H-NMR ($CDCl_3$) $\delta$ = 9,45 (d, 1H); 7,3-7,0 (m, 9H); 5,25 (t, 1H); 4,7-4,3 (m, 3H); 3,9 (m, 1H); 3,8-3,55 (m, 2H); 3,3-3,0 (m, 2H); 2,7 (t, 2H); 2,5 (m, 2H); 2,1-1,8 (m, 8H)ppm.

**Beispiel 16**

N-(4-Phenylbutyryl)-cis,exo-octahydroindol-2-carbonsäurepyrrolidid

a) N-(4-Phenylbutyryl)-cis, exo-ootahydroindol-2-carbonsäurebenzylester

2,5 g (9,6 mmol) cis, exo-Octahydroindol-2-carbonsäurebenzylester werden zusammen mit 1,7 g 4-Phenylbuttersäure, 1,4 ml N-Ethylmorpholin 1,6 g 1-Hydroxybenzotriazol in 80 ml trockenen Dimethylformamid gelöst. Nach Zugabe von 2,2 g Dicyclohexylcarbodiimid bei 0°C wird 18 Stunden bei Raumtemperatur gerührt. Nach Absaugen des gebildeten Niederschlags wird mit Essigester verdünnt und jeweils 1 mal mit 10 % Citronensäure, 3 % $NaHCO_3$-Lösung und gesättigter Kochsalzlösung gewaschen, mit Magnesiumsulfat getrocknet und eingeengt. Chromatographie an Kieselgel (Ethylacetat/Cyclohexan (1:2) ergibt 2,2 g der Titelverbindung als Öl.

$^1$H-NMR ($CDCl_3$) $\delta$ = 7,4-7,1 (m, 10H); 5,15 (dd, 2H); 4,5 (d, 1H); 3,7 (m, 1H); 2,65 (t, 2H); 2,6 (m, 1H); 2,4-2,2 (m, 2H); 2,1-1,1 (m, 10H)ppm.

b) N-(4-Phenylbutyryl)-cis, exo-octahydroindol-2-carbonsäure

2,2 g der Verbindung aus Beispiel 16a) werden in 60 ml Ethanol mit 0,2 g Pd/C (10 %) als Katalysator bei 1,2 bar und Raumtemperatur hydriert. Nach Ende der $H_2$-Aufnahme wird der Katalysator abfiltriert und die Lösung eingeengt. Man erhält 1,5 g farblose Kristalle von Schmp. 114°C.

c) N-(4-Phenylbutyryl)-cis, exo-octahydroindol-2-carbonsäurepyrrolidid

Analog den in Beispiel 13c) angegebenen Verfahren erhält man aus 0,75 g der Verbindung aus Beispiel 16b), 0,23 ml Pyrrolidin, 0,33 ml N-Ethylmorpholin, 0,37 g 1-Hydroxybenzotriazol und 0,49 g Dicyclohexylcarbodiimid nach Chromatographie an Kieselgel (Laufmittel Ethylacetat) 0,27 g der Titelverbindung als Öl.

$^1$H-NMR ($CDCl_3$) $\delta$ = 7,3-7,1 (m, 5H), 4,6 (d, 1H); 3,8 (m, 2H); 3,6 (m, 1H); 3,4 (m, 2H); 2,9 (m, 1H); 2,7 (t, 2H); 2,5-1,1 (m, 16H)ppm.

**Beispiel 17**

N-[N-(4-Phenylbutyryl)-cis, exo-octahydroindol-2-carbonyl]-S-prolinol

Analog dem in Beispiel 14 angebenen Verfahren erhält man aus 0,75 g (2,38 mmol) der Verbindung aus Beispiel 16b) sowie 0,23 ml S-Prolinol, 0,33 ml N-Ethylmorpholin, 0,37 g 1-Hydroxybenzotriazol und 0,49 g Dicyclohexylcarbodiimid 0,6 g der Titelverbindung nach Chromatographie an Kieselgel mit Ethylacetat als Laufmittel.

$^1$H-NMR ($CDCl_3$) $\delta$ = 7,3-7,1 (m, 5H); 4,6 (dd, 1H); 4,4-3,9 (m, 3H); 3,8-3,4 (m, 4H); 2,9 (m, 2H); 2,7 (m, 2H); 2,4-1,1 (m, 16H)ppm.

**Beispiel 18**

N-[N-(4-Phenylbutyryl)-cis, exo-octahydroindol-2-carbonyl]-S-prolinal

Analog den in Beispiel 2 angegebenen Verfahren erhält man aus 0,6 g der Verbindung aus Beispiel 17 und 0,25 ml Oxalylchlorid, 0,4 ml DMSO und 1,6 ml Triethylamin nach Chromatographie an Kieselgel mit Dichlormethan/Methanol (40:1) als Laufmittel 0,43 g der Titelverbindung als Öl.

$^1$H-NMR ($CDCl_3$) $\delta$ = 9,6-9,4 (m, 1H); 7,3-7,15 (m, 5H); 4,6 (m, 1H); 4,3-3,4 (m, 4H); 2,9 (m, 1H); 2,7 (m, 2H); 2,5-1,1 (m, 16H)ppm.

**Beispiel 19**

N-[4-(2-Benzyl-phenoxy)-butyryl]-(2S,3aS,6aS)-octahydrocyclopenta[b]pyrrol-2-carbonsäure-pyrrolidid

a) N-[4-(2-Benzyl-phenoxy)-butyryl]-(2S,3aS,6aS)-octahydrocyclopenta[b]pyrrol-2-carbonsäure-benzylester

Nach dem in Beispiel 1a) angegebenen Verfahren erhält man aus 9,6 g (35 mmol) 4-(2-Benzyl-phenoxy)-buttersäure und 17,4 g (71 mmol) (2S,3aS,6aS)-Octahydrocyclopena[b]pyrrol-2-carbonsäure-benzylester unter Verwendung von 5,6 ml Chlorameisensäureethylester und 15,8 ml Triethylamin nach Chromatographie an Kieselgel (Laufmittel Ethylacetat/Cyclohexan (1:2)) 8,8 g der Titelverbindung als Öl.

$^1$H-NMR ($CDCl_3$) $\delta$ = 7,4-6,9 (m, 14H), 5,1 (s, 2H); 4,8-3,9 (m, 6H); 3,0-1,0 (m, 13H)ppm.

b) N-[4-(2-Benzyl-phenoxy)-butyryl]-(2S,3aS,6aS)-octahydrocyclopenta[b]pyrrol-2-carbonsäure

Nach dem in Beispiel 16b) angegebenen Verfahren erhält man aus 8,8 g der Verbindung aus Beispiel 19a) 7,6 g der Titelverbindung als Öl.

$^1$H-NMR ($CDCl_3$) $\delta$ = 7,4-6,9 (m, 14H), 5,1 (s, 2H); 4,8-3,9 (m, 6H); 3,0-1,0 (m, 13H)ppm.

c) N-[4-(2-Benzyl-phenoxy)-butyryl]-(2S,3aS,6aS)-octahydrocyclopenta[b]pyrrol-2-carbonsäure-pyrrolidid

Nach dem in Beispiel 13c) angegebenen Verfahren erhält man aus 2,5 g der Verbindung aus Beispiel 19b) 0,7 ml Pyrrolidin, 1 ml N-Ethylmorpholin, 1,1 g 1-Hydroxybenzotriazol und 1,5 g Dicyclohexylcarbodiimid 1,6 g der Titelverbindung nach Chromatographie an Kieselgel (Laufmittel Ethylacetat/Methanol 20:1).

$^1$H-NMR ($CDCl_3$) $\delta$ = 7,3-7,1 (m, 7H); 6,85 (t, 3H); 4,63 (dd, 1H); 4,1-3,9 (m, 5H); 3,8 (m, 1H); 3,6 (m, 1H); 3,4 (m, 2H); 2,75 (m, 1H); 2,5-1,5 (m, 14H)ppm.

**Beispiel 20**

N-[N[4-(2-Benzyl-phenoxy)-butyryl]-(2S,3aS,6aS)-octahydrocyclopenta[b]pyrrol-2-carbonyl]-S-prolinol

Analog dem in Beispiel 14 angegebenen Verfahren erhält man aus 5 g der Verbindung aus Beispiel 19b) und 1,53 ml S-Prolinol, 2,2 ml N-Ethylmorpholin, 2,46 g 1-Hydroxybenzotriazol sowie 3,26 g Dicyclohexylcarbodiimid 3,8 g der Titelverbindung als Öl nach Chromatographie an Kieselgel.

$^1$H-NMR ($CDCl_3$) δ 7,3-7,1 (m, 7H); 6,85 (m, 2H); 5,15+4,65 (2t, 1H); 4,9-3,4 (m, 6H); 2,7 (m, 1H); 2,5-1,5 (m, 16H)ppm.

**Beispiel 21**

N-[N-[4-(2-Benzyl-phenoxy)-butyryl]-(2S,3aS,6aS)-octahydrocyclopenta[b]pyrrol-2-carbonyl]-S-prolinal

Analog dem in Beispiel 15 angegebenen Verfahren erhält man aus 3,38 g der Verbindung aus Beispiel 20), 1,3 ml Oxalylchlorid, 2,7 ml DMSO und 8,2 ml Triethylamin 2,7 g der Titelverbindung als Öl nach Chromatographie an Kieselgel mit Ethylacetat/Methanol (20:1) als Laufmittel.

$^1$H-NMR ($CDCl_3$) δ = 9,5 (s, 1H); 7,3-7,1 (m, 7H); 6,85 (m, 2H); 4,7 (m, 2H); 4,1-3,4 (m, 3H); 2,7 (m, 1H); 2,45 (m, 3H); 2,3-1,5 (m, 14H)ppm.

**Beispiel 22**

3-[N-[N-(4-Phenylbutyryl)-(2S,3aS,6aS)-octahydrocyclopenta[b]-pyrrol-2-carbonyl]-2S-pyrrolidinyl]-acrylsäuremethylester

1,1 g (6 mmol) Trimethylphosphonoacetat werden in 20 ml THF gelöst und bei -30 °C mit 0,67 g Kalium-tert.butylat versetzt. Nach 30 Minuten bei Raumtemperatur werden bei -78 °C 2,2 g (6 mmol) der Verbindung aus Beispiel 2 zugesetzt. Nach langsamen Aufwärmen auf Raumtemperatur und 30 minutigem Rühren wird mit Wasser verdünnt und mit Essigester extrahiert. Die vereinigten Extrakte werden mit gesättigter wäßriger Kochsalzlösung gewaschen, über Natriumsulfat getrocknet und eingeengt. Chromatographie an Kieselgel (Ethylacetat/Cyclohexan 1:1) ergibt 1,9 g der Titelverbindung als farbloses Öl.

$^1$H-NMR ($CDCl_3$) δ = 7,2 (s, 5H); 7,05-6,6 (m, 1H); 6,1-5,6 (m, 1H); 4,9-4,2 (m, 2H); 3,8-3,6 (m, 1H); 3,7 (s, 3H); 3,6-3,2 (m, 2H); 2,8-2,4 (m, 5H); 2,4-1,6 (m, 10H)ppm.

**Beispiel 23**

3-[N-[N-(4-Phenylbutyryl)-(2S,3aS,6aS)-octahydrocyclopenta[b]-pyrrol-2-carbonyl]-2S-pyrrolidinyl]-acrylsäure

1,2 g der Verbindung aus Beispiel 22 werden in 10 ml Methanol gelöst und 16 Stunden mit 4,5 ml 1 N wäßriger NaOH gerührt. Das Methanol wird im Vakuum entfernt, der Rückstand mit Wasser verdünnt und mit Essigester extrahiert. Die wäßrige Phase wird mit 2 N HCl angerührt und mit Essigester extrahiert. Nach Trocknen der organischen Phase über $MgSO_4$ wird eingeengt. Das Rohprodukt wird an Kieselgel chromatographiert (Laufmittel Dichlormethan/Methanol (10:1). Man erhält 0,8 g der Titelverbindung als farbloses Öl.

$^1$H-NMR ($CDCl_3$) δ = 7,2 (s, 5H); 7,05-6,6 (m, 1H); 6,1-5,6 (m, 1H); 4,9-4,2 (m, 2H); 3,8-3,6 (m, 1H); 3,6-3,2 (m, 2H); 2,8-2,4 (m, 5H); 2,4-1,6 (m, 10H)ppm.

**Beispiel 24**

3-[N-N-(4-Phenylbutyryl)-(2S,3aS,6aS)-octahydrocyclopenta[b]-pyrrol-2-carbonyl]-2S-pyrrolidinyl]-acrylsäure-N-(4-methyl-piperazid)

1,42 g der Verbindung aus Beispiel 23 werden in 20 ml DMF nacheinander mit 0,53 g 1-Hydroxybenzotriazol, 0,4 g N-Ethylmorpholin, 0,39 g N-Methylpiperazin und 0,72 g Dicyclohexylcarbodiimid versetzt. Die Aufarbeitung erfolgt wie in Beispiel 1b) angegeben. Nach Chromatographie an Kieselgel (Dichlormethan/Methanol (20:1)) erhält man 0,75 g der Titelverbindung als Öl.

$^1$H-NMR ($CDCl_3$) $\delta$ = 7,2 (s, 5H); 7,0-6,0 (m, 2H); 4,8-4,3 (m, 2H); 3,8-3,3 (m, 7H); 2,9-1,4 (m, 23H); 2,3 (s, 3H)ppm.

**Beispiel 25**

N-[N-(4-Phenylbutyryl)-(2S,3aS,6aS)-octahydrocyclopenta[b]-pyrrol-2-carbonsäure]-(2S-trifluoroacetyl)-pyrrolidid

a) N-[N-(-Phenylbutyryl)-(2S,3aS,6aS)-octahydrocyclopenta-[b]pyrrol-2S-carbonsäure]-[2S-(1-hydroxy-2,2,2-trifluorethyl)-pyrrolidid]

In eine Mischung aus 1,8 g der Verbindung aus Beispiel 2 und 0,65 g Zinkpulver in 15 ml Dimethylformamid werden bei 20 °C 10 g Trifluorjodmethan unter Beschallung im Ultraschallbad eingeleitet. Nach 5 Stunden wird mit 100 ml 0,1 N Salzsäure versetzt, mit Ethylacetat extrahiert, die vereinigten Essigesterphasen dreimal mit Wasser gewaschen, mit $MgSO_4$ getrocknet und eingeengt. Chromatographie an Kieselgel (Ethylacetat/Cyclohexan (5:1)) ergibt 0,7 g der Titelverbindung als Öl.

$^1$H-NMR ($CDCl_3$) $\delta$ = 7,4-7,2 (m, 5H); 5,0 (dd, 1H); 4,7 (m, 1H); 4,2-3,8 (m, 2H); 3,8 (m, 1H); 3,5 (m, 1H); 2,8-2,6 (m, 3H); 2,4-2,2 (m, 3H); 2,2-1,5 (m, 10H)ppm

b)N-[N-(4-Phenylbutyryl)-(2S,3aS,6aS)-octahydrocyclopenta[b]-pyrrol-2S-carbonsäure]-(2S-trifluoracetyl)-pyrrolidid

Analog der in Beispiel 2 angegebenen Verschrift erhält man aus 0,7 g der Verbindung aus Beispiel 25a) nach Chromatographie an Kieselgel 0,48 g der Titelverbindung als Öl.

$^1$H-NMR ($CDCl_3$) $\delta$ = 7,4-7,2 (m, 5H), 5,0 (dd, 1H); 4,7 (m, 1H); 4,15 (m, 1H); 3,8 (m, 1H); 3,5 (m, 1H); 2,8-2,6 (m, 3H); 2,4-2,2 (m, 3H); 2,2-1,5 (m, 10H)ppm.

**Beispiel 26**

1-[N-[N-(4-Phenylbutyryl)-(2S,3aS,6aS)-octahydrocyclopenta[b]-pyrrol-2-carbonyl]-2S-pyrrolidinyl]-2,2-difluor-6-phenyl-hexandion-1,3

a) 3-[N-[N-(4-Phenylbutyryl)-(2S,3aS,6aS)-octahydrocyclopenta[b]pyrrol-2S-carbonyl]-2S-pyrrolidinyl]-2,2-difluor-3-hydroxy-propionsäure

1,2 g der Verbindung aus Beispiel 3 werden mit 10 ml 1 N NaOH in 10 ml Methanol 10 Stunden bei Raumtemperatur gerührt. Nach Entfernen des Methanols wird, wie in Beispiel 23 beschrieben, aufgearbeitet. Man erhält 0,9 g der Titelverbindung als Öl.

$^1$H-NMR ($CDCl_3$) $\delta$ = 7,3-7,1 (m, 5H); 5,0 (dd, 1H); 4,75-4,6 (m, 2H); 4,3-3,6 (m, 5H); 3,5 (m, 1H); 2,8 (m, 1H); 2,65 (2t, 2H); 2,4-2,2 (m, 3H); 2,15-1,5 (m, 12H)ppm.

b) 3-N-[N-(4-Phenylbutyryl)-(2S,3aS,6aS)-octahydrocyclopenta[b]pyrrol-2S-carbonyl]-2S-pyrrolidonyl]-2,2-difluor-3-hydroxy-propionsäure-N-methyl-N-methoxy-amid

0,9 g (1,9 mmol) der Verbindung aus Beispiel, 26a) werden zusammen mit 0,18 g N, O-Dimethylhydroxylamin . Hydrochlorid, 0,26 g 1-Hydroxybenzotriazol und 0,22 g N-Ethylmorpholin in 10 ml Dimethylformamid gelöst. Nach Zugabe von 0,39 g Dicyclohexylcarbodiimid wird 20 Stunden bei Raumtemperatur gerührt und wie in Beispiel 1b) beschrieben aufgearbeitet.

Chromatographie an Kieselgel ergibt 0,86 g der Titelverbindung als Öl.

$^1$H-NMR ($CDCl_3$) $\delta$ =7,3-7,1 (m, 5H); 5,3-5,0 (m, 1H); 4,75 -4,6 (m, 2H); 4,3-3,6 (m, 5H); 3,9 (s, 3H); 3,5 (m, 1H); 3,3 (s, 3H); 2,8 (m, 1H); 2,65 (2t, 2H); 2,4-2,2 (m, 3H); 2,15-1,5 (m, 12H)ppm.

c) 1-[N-[N-(4-Phenylbutyryl)-(2S,3aS,6aS)-octahydrocyclopenta[b]pyrrol-2S-carbonyl]-2S-pyrrolidinyl]-2,2-difluor-1-hydroxy-6-phenyl-hexanon-3

1,63 g (8,2 mmol) 3-Phenylpropylbromid werden bei Raumtemperatur zu 200 mg Magnesiumspänen in 20 ml THF gegeben und bis zur Auflösung des Metalls am Rückfluß gekocht. Anschließend tropft man bei 0 °C eine Lösung von 0,86 g der Verbinding aus Beispiel 26b) in 5 ml THF zu und rührt noch 5 Stunden bei Raumtemperatur. Nach Zugabe von 2 ml 1 N HCl wird mit Ethylacetat/Wasser verdünnt und die organische Phase abgetrennt. Nach Waschen mit gesättigter Kochsalzlösung wird mit $MgSO_4$ getrocknet und eingeengt. Chromatographie an Kieselgel ergibt 0,7 g der Titelverbindung als Öl.

$^1$H-NMR ($CDCl_3$) $\delta$ = 7,3-7,0 (m, 10H); 5,3-4,9 (m, 1H); 4,7-4,4 (m, 2H); 4,3-3,6 (m, 5H); 3,5 (m, 1H); 2,8(m, 1H); 2,9-2,5 (m, 4H); 2,4-2,2 (m, 5H); 2,15-2,5 (m, 14H)ppm.

d) 1-[N-[N-(4-Phenylbutyryl)-(2S,3aS,6aS)-octahydrocyclopenta[b]pyrrol-2-carbonyl]-2S-pyrrolidinyl]-2,2-difluor-6-phenyl-hexandion-1,3

Eine Lösung von 5,08 g (12 mmol)1,1-Dihydro-1,1,1-triacetoxy-1,2-benzjodoxol-3(1H)-on in 12 ml Dichlormethan wird zu einer gerührten Lösung von 0,7 g der Verbindung aus Beispiel 26c) in 40 ml Dichlormethan gegeben. 1,4 g Trifluoressigsäure werden zugegeben und 13 Stunden bei Raumtemperatur gerührt. Nach Verdünnen mit Ethylacetat wird mit gesättigter Natriumthiosulfat-, gesättigter Natriumbicarbonat- und gesättigter Kochsalzlösung gewaschen mit $MgSO_4$ getrocknet und eingeengt. Chromatographie ergibt 0,5 g der Titelverbindung als Öl.

$^1$H-NMR ($CDCl_3$) $\delta$ = 7,4-7,1 (m, 10H), 5,0 (dd, 1H); 4,7 (m, 1H); 4,2 (m, 1H); 3,85 (m, 1H); 3,5 (m, 1H); 2,8-2,6 (m, 5H); 2,4-2,2 (m, 5H); 2,2-1,5 (m, 12H)ppm.

**Beispiel 27**

2-[N-[N-(4-Phenylbutyryl)-(2S,3aS,6aS)-octahydrocyclopenta[b]-pyrrol-2-carbonyl]-2S-pyrrolidinyl]-2-oxo-essigsäureethylester

a) N-[N-(4-Phenylbutyryl)-(2S,3aS,6aS)-octahydrocyclopenta[b]-pyrrol-2-carbonyl]-2-(2-methylthio-2-methylsulfoxyacetyl)-pyrrolidin

Zu einer Suspension von Natriumhydrid (50 % in Öl; 0,3 g) in 10 ml Dimethoxyethan gibt 0,8 g Methyl-methylsulfinylmethyl sulfid und rührt 2 Stunden bei 50 °C. Anschließen gibt man 0,8 g N-[N-(4-Phenylbutyryl)-(2S,3aS,6aS)-octahydrocyclopenta[b]pyrrol]-2-carbonyl-S-prolin-methylester unter Kühlung zu und rührt 15 Stunden bei Raumtemperatur. Anschließend gießt man in Eiswasser und extrahiert mit Ethylacetat. Nach Trocknen mit $MgSO_4$ und Einengen erhält man 0,6 g der Titelverbindung nach Chromatographie an Kieselgel.

$^1$H-NMR ($CDCl_3$) $\delta$ = 7,5-7,2 (m, 5H); 5,0-4,8 (m, 1H); 4,7 (s, 1H); 4,6 (m, 1H); 4,2 (m, 1H); 3,8 (m, 1H); 3,5 (m, 1H); 2,8-2,6 (m, 3H); 2,8 (s, 3H); 2,2 (s, 3H); 2,4-2,2 (m, 3H); 2,2-1,5 (m, 10H)ppm.

b) 2-[N-[N-(4-Phenylbutyryl)-(2S,3aS,6aS)-octahydrocyclopenta[b]pyrrol-2-carbonyl]-2S-pyrrolidinyl]-2-oxo-essigsäureethylester

Eine Mischung aus 0,6 g der Verbindung aus Beispiel 27a) und 0,2 g Kupfer(II)chlorid-Dihydrat in 5 ml Ethanol wird 15 Stunden bei Raumtemperatur gerührt. Nach Einengen wird mit Ethylacetat aufgenommen,

mit Wasser gewaschen, über $MgSO_4$ getrocknet und eingeengt. Chromatographie an Kieselgel mit Essigester als Laufmittel ergibt 0,4 g der Titelverbindung als Öl.

$^1$H-NMR ($CDCl_3$) $\delta$ = 7,4-7,2 (m, 5H); 5,0 (dd, 1H); 4,7 (m, 1H); 4,35-4,0 (m, 3H); 3,8 (m, 1H); 3,5 (m, 1H); 2,8-2,6 (m, 3H); 2,4-2,2 (m, 3H); 2,2-1,5 (m, 10H); 1,3 (t, 3H)ppm.

**Beispiel 28**

2-[N-[N-(4-Phenylbutyryl)-(2S,3aS,6aS)-octahydrocyclopenta[b]-pyrrol-2-carbonyl]-2S-pyrrolidinyl]-2-oxo-essigsäure-N-benzylamid

0,6 g der Verbindung aus Beispiel 27b) werden analog der in Beispiel 23 angegebenen Vorschrift zur Säure hydrolysiert. Die rohe Säure wird nach der in Beispiel 16 angegebenen Vorschrift mit Benzylamin umgesetzt. Man erhält nach Chromatographie an Keiselgel 0,37 g der Titelverbindung als Öl.

$^1$H-NMR ($CDCl_3$) $\delta$ = 7,4-7,1 (m, 10H), 5,0-4,8 (m, 1H); 4,7 (m, 1H); 4,2 (m, 1H); 3,8 (m, 1H); 3,5 (m, 3H); 2,8-2,5 (m, 3H); 2,4-2,2 (m, 3H); 2,2-1,5 (m, 10H)ppm.

Analog den in den vorstehenden Beispielen beschriebenen Verfahren wurden unter Verwendung entsprechender Ausgangsstoffe weiterhin hergestellt:

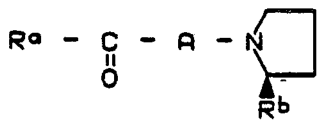

| Bsp.-Nr. | $R^a$ | A | $R^b$ |
|---|---|---|---|
| 29 | $C_6H_5(CH_2)_3$- | 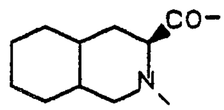 | H |
| 30 | $C_6H_5(CH_2)_3$- | 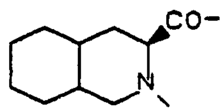 | CHO |
| 31 | 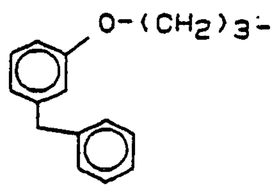 | 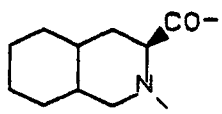 | H |
| 32 | $C_6H_5(CH_2)_3$- | 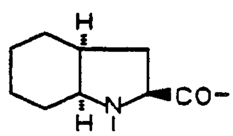 | H |
| 33 | $C_6H_5(CH_2)_3$- | 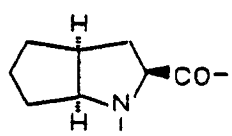 | H |
| 34 | $C_6H_5(CH_2)_3$- | 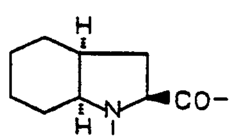 | CHO |
| 35 | 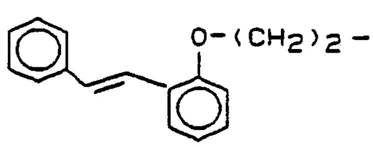 | 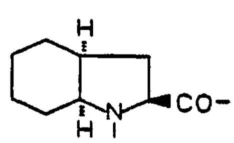 | H |

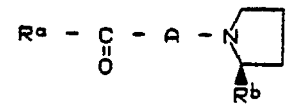

| Bsp.-Nr. | $R^a$ | A | $R^b$ |
|---|---|---|---|
| 36 | 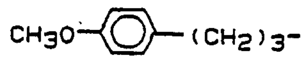 | 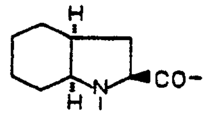 | $CO-CF_3$ |
| 37 | 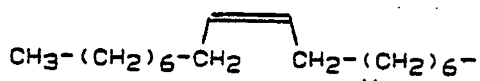 | 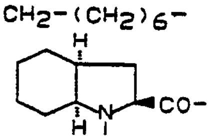 | H |
| 38 | 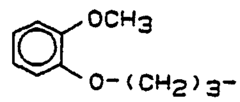 | 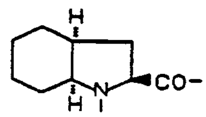 | $COCF_2CONHCH_2C_6H_5$ |
| 39 | $C_6H_5(CH_2)_3-$ | 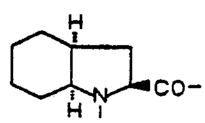 | 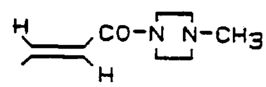 |
| 40 | $C_6H_5-(CH_2)_3-$ | 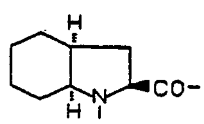 | $-CO-CO_2CH_3$ |
| 41 | 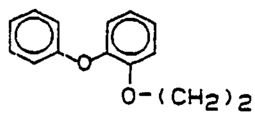 | 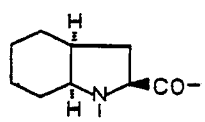 | $-CO-CF_2-COOCH_3$ |
| 42 | 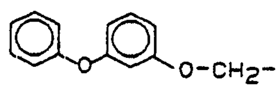 | 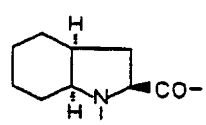 | H |
| 43 | $C_6H_5(CH_2)_3-$ | 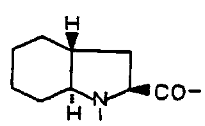 | H |

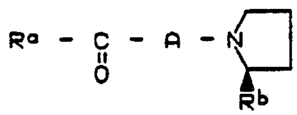

| Bsp.-Nr. | $R^a$ | A | $R^b$ |
|---|---|---|---|
| 44 | 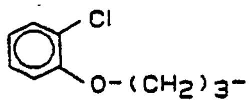 | 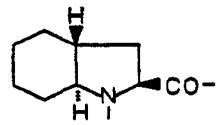 | CHO |
| 45 | 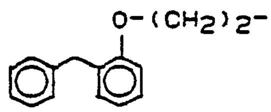 | 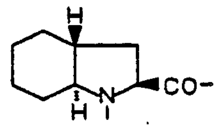 | H |
| 46 | 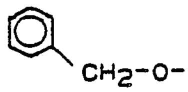 | 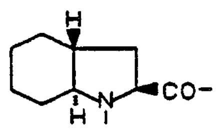 | CHO |
| 47 | 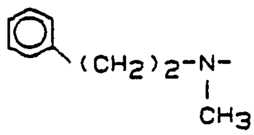 | 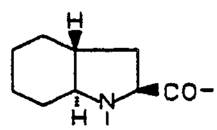 | H |
| 48 | 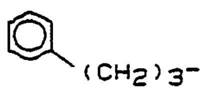 | 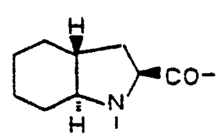 | $COCF_2CONHCH_2C_6H_5$ |
| 49 | $C_6H_5-(CH_2)_3-$ | 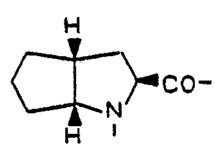 | H |
| 50 | 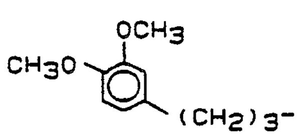 | 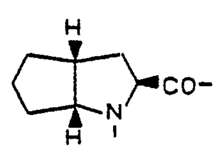 | CHO |

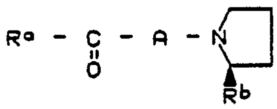

| Bsp.-Nr. | $R^a$ | A | $R^b$ |
|---|---|---|---|
| 51 | $C_6H_5CH_2O-$ | 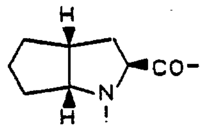 | $COCF_2CONH(CH_2)_2C_6H_5$ |
| 52 | $C_6H_5CH_2O-$ | 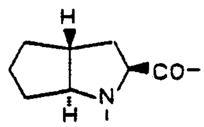 | H |
| 53 | $C_6H_5(CH_2)_3-$ | 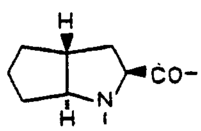 | CHO |
| 54 | $C_6H_5-(CH_2)_3-$ | 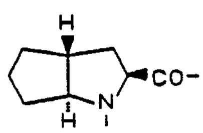 | $COCF_2CONHCH_2-$ 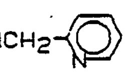 |
| 55 | $C_6H_5-(CH_2)_3-$ | 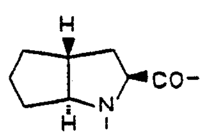 | $COCF_2(CH_2)_3C_6H_5$ |
| 56 | $C_6H_5-(CH_2)_4-$ | 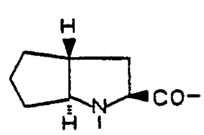 | $-CH=CH-$ 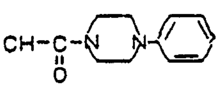 |
| 57 | 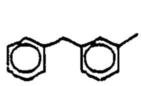 $O(CH_2)_2-$ | 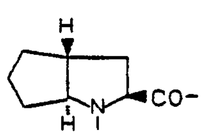 | H |
| 58 | $(C_6H_5)_2CHCH_2-$ | 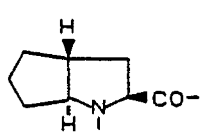 | CHO |

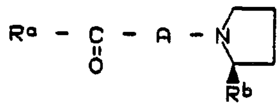

| Bsp.-Nr. | $R^a$ | A | $R^b$ |
|---|---|---|---|
| 59 | $C_6H_5-CH_2-CH(CH_3)-$ | 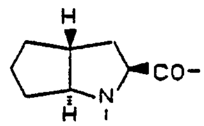 | CHO |
| 60 | $C_6H_5-(CH_2)_3-$ | 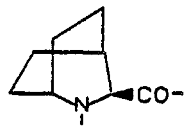 | H |
| 61 | $C_6H_5-(CH_2)_3-$ | 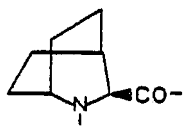 | CHO |
| 62 | $C_6H_5-(CH_2)_3-$ | 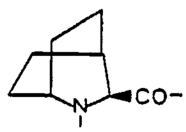 | $COCF_2CONH-(CH_2)_2C_6H_5$ |
| 63 | $C_6H_5-(CH_2)_3-$ | 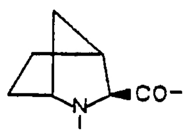 | H |
| 64 | $C_6H_5-CH_2-O-$ | 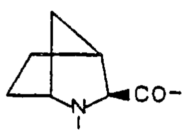 | CHO |
| 65 | $CH_3O-$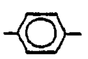$-(CH_2)_3-$ | 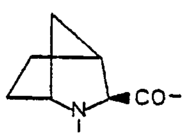 | $COCF_2COOCH_3$ |
| 66 | $C_6H_5-(CH_2)_3-$ | 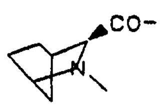 | H |

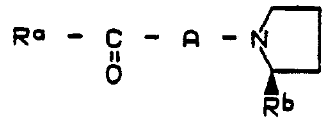

| Bsp.-Nr. | $R^a$ | A | $R^b$ |
|---|---|---|---|
| 67 | $C_6H_5-(CH_2)_3-$ | 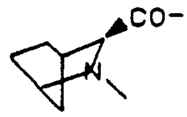 | CHO |
| 68 | $C_6H_5-(CH_2)_3-$ | 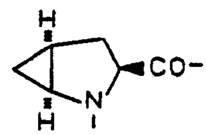 | CHO |
| 69 | 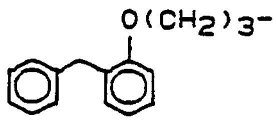 | 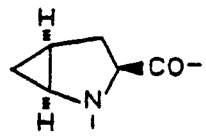 | H |
| 70 | $C_6H_5-(CH_2)_3-$ | 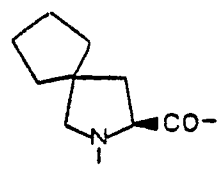 | CHO |
| 71 | 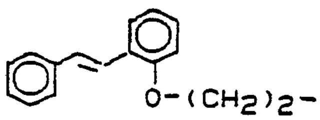 | 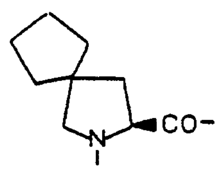 | H |
| 72 | 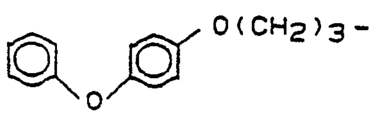 | 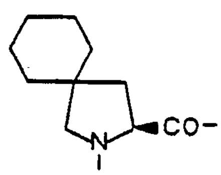 | H |
| 73 | $C_6H_5-(CH_2)_3-$ | 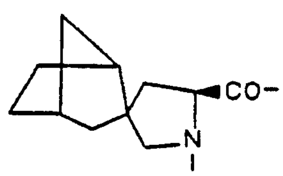 | H |

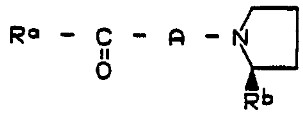

| Bsp.-Nr. | $R^a$ | A | $R^b$ |
|---|---|---|---|
| 74 | $C_6H_5-(CH_2)_3-$ | 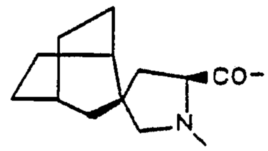 | H |
| 75 | $C_6H_5-(CH_2)_3-$ | 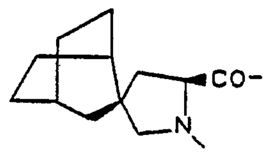 | CHO |
| 76 | $C_6H_5-CH_2O-$ | 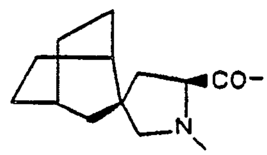 | $CH_3$<br>$COCF_2CON-CH-C_6H_5$<br>H |
| 77 | $CH_3O-$ 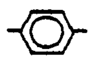 $-(CH_2)_2-$ | 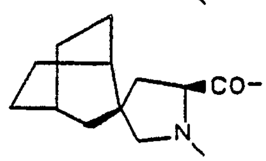 | $COCO_2C_2H_5$ |
| 78 | $C_6H_5-(CH_2)_3-$ | 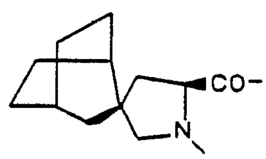 | $COCF_3$ |
| 79 | $C_6H_5-(CH_2)_3-$ | 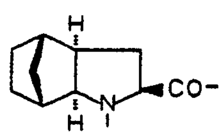 | CHO |
| 80 | 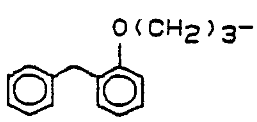 | 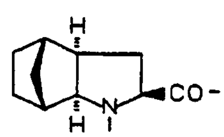 | H |
| 81 | $C_6H_5-(CH_2)_3-$ | 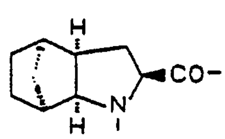 | H |

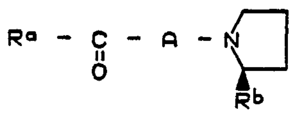

| Bsp.-Nr. | $R^a$ | A | $R^b$ |
|---|---|---|---|
| 82 | $C_6H_5-(CH_2)_3-$ | 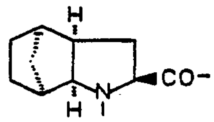 | CHO |
| 83 | $C_6H_5-(CH_2)_3-$ | 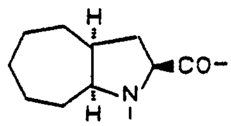 | H |
| 84 | $C_6H_5-(CH_2)_3-$ | 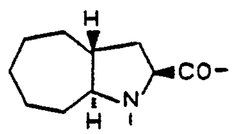 | CHO |
| 85 | $C_6H_5-(CH_2)_3-$ | 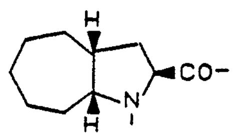 | H |
| 86 | $C_6H_5(CH_2)_3-$ | 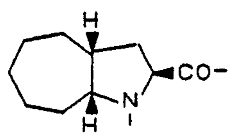 | CHO |
| 87 | $C_6H_5-(CH_2)_3-$ | 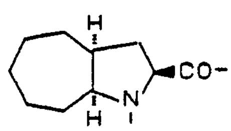 | CHO |
| 88 | $C_6H_5-(CH_2)_3-$ | 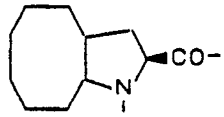 | CHO |
| 89 | $C_6H_5-(CH_2)_3-$ | 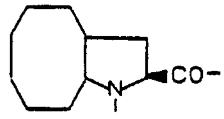 | $COCF_2CONH-(CH_2)_2C_6H_5$ |

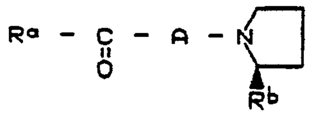

| Bsp.-Nr. | $R^a$ | A | $R^b$ |
|---|---|---|---|
| 90 | $C_6H_5-(CH_2)_3-$ | 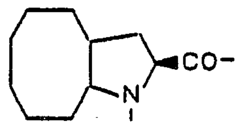 | H |
| 91 | $C_6H_5-(CH_2)_3-$ | 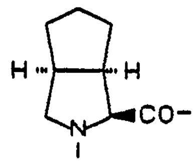 | H |
| 92 | $C_6H_5-(CH_2)_3-$ | 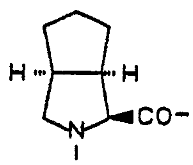 | CHO |
| 93 | 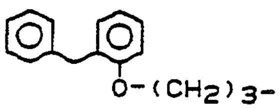 | 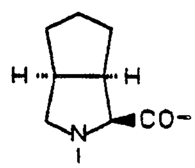 | H |
| 94 | $C_6H_5-(CH_2)_3-$ | 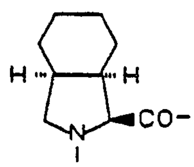 | H |
| 95 | $C_6H_5-(CH_2)_3-$ | 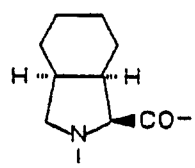 | CHO |
| 96 | $C_6H_5-(CH_2)_3-$ | 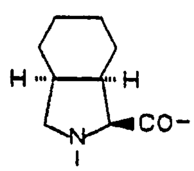 | $COCF_2CONHCH_2C_6H_5$ |
| 97 | $C_6H_5CH_2O-$ | 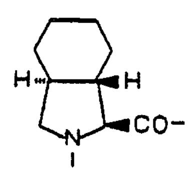 | H |

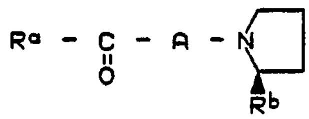

| Bsp.-Nr. | $R^a$ | A | $R^b$ |
|---|---|---|---|
| 98 | $C_6H_5-(CH_2)_3-$ | 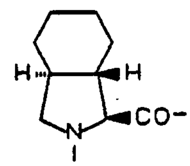 | CHO |
| 99 | $C_6H_5-(CH_2)_3-$ | 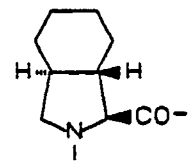 | $COCF_3$ |
| 100 | $C_6H_5-(CH_2)_3-$ | 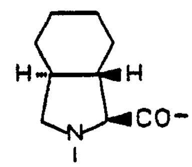 | $COCO\underset{H}{N}CH_2C_6H_5$ |
| 101 | $C_6H_5(CH_2)_3-$ | 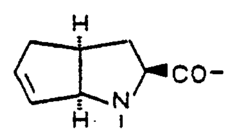 | H |
| 102 | $C_6H_5(CH_2)_3-$ | 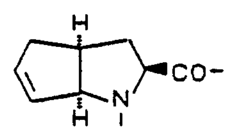 | CHO |
| 103 | $C_6H_5-(CH_2)_3-$ | 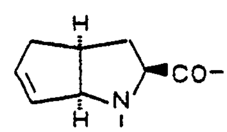 | 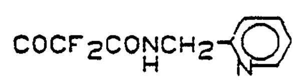 |
| 104 | $C_6H_5(CH_2)_3-$ | 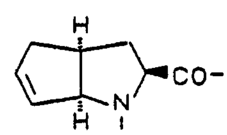 | 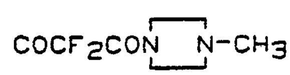 |
| 105 | $C_6H_5(CH_2)_3-$ | 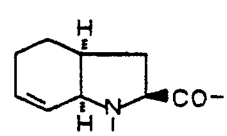 | CHO |

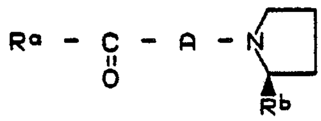

| Bsp.-Nr. | $R^a$ | A | $R^b$ |
|---|---|---|---|
| 106 | $C_6H_5-CH_2-O-$ | 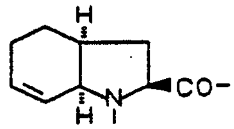 | H |
| 107 | 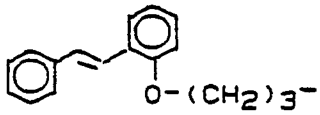 | 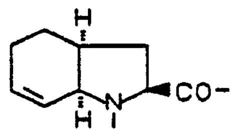 | H |
| 108 | 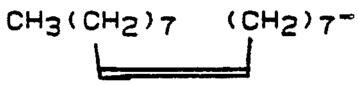 | 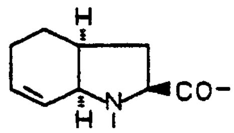 | H |
| 109 | 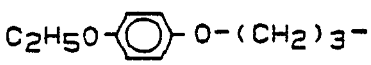 | 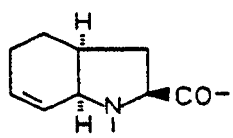 | CHO |
| 110 | $C_6H_5(CH_2)_3-$ | 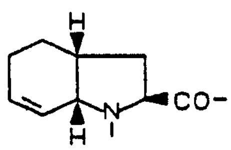 | H |
| 111 | $C_6H_5(CH_2)_3-$ | 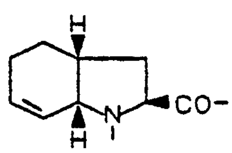 | CHO |
| 112 | $C_6H_5(CH_2)_3-$ | 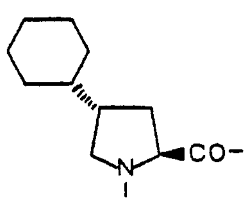 | H |

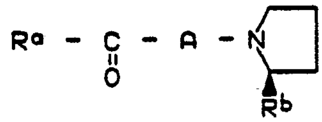

| Bsp.-Nr. | $R^a$ | A | $R^b$ |
|---|---|---|---|
| 113 | $C_6H_5(CH_2)_3-$ | 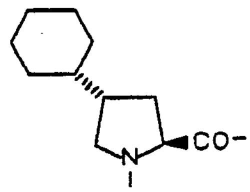 | CHO |
| 114 | $C_6H_5-(CH_2)_3-$ | 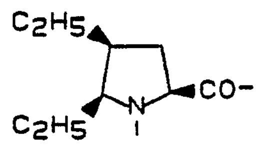 | H |
| 115 | $C_6H_5-(CH_2)_3-$ | 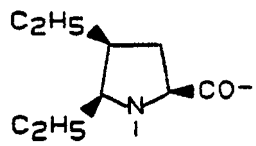 | CHO |
| 116 | $C_6H_5-(CH_2)_3-$ | 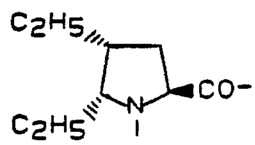 | H |
| 117 | $C_6H_5-(CH_2)_3-$ | 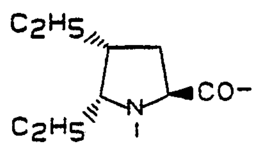 | CHO |
| 118 | $C_6H_5-(CH_2)_3-$ | 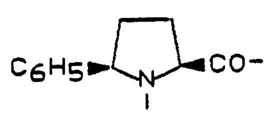 | H |
| 119 | $C_6H_5-(CH_2)_3-$ | 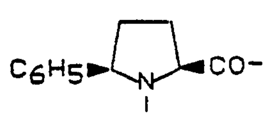 | CHO |
| 120 | $C_6H_5-(CH_2)_3-$ | 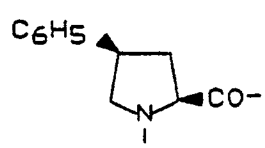 | H |

**Beispiel 121**

N-(Cinnamoyl)-(2S,3aS,6aS)-octahydrocyclopenta[b]pyrrol-2 carbonsäure-pyrrolidid

0,5 g (2,4 mmol) (2S,3aS,6aS)-Octahydrocyclopenta[b]pyrrol-2-carbonsäure-pyrrolidid und 0,42 g trans-Zimtsäure werden zusammen mit 1,5 ml N-Ethylmorpholin in 20 ml trockenem Dimethylformamid bei 0° C mit 1,5 ml n-Propanphosphorsäureanhydrid (50 % in $CH_2Cl_2$) versetzt. Nach 8 Stunden bei Raumtemperatur wird mit Ethylacetat verdünnt, je 1 mal mit 3 % $NaHCO_3$-Lösung, 10 % Citronensäure, 3 % $NaHCO_3$-Lösung und gesättigter Kochsalzlösung gewashen, mit $MgSO_4$ getrocknet und eingeengt. Nach Chromatographie an Kieselgel (Laufmittel Ethylacetat/Methanol = 10:1) erhält man 0,4 g der Titelverbindung.
Schmp. 176° C
$[\alpha]_D^{20}$ = + 111,5° (C = 0,438; $CH_2Cl_2$).

a) (2S,3aS,6aS)-Octahydrocyclopenta[b]pyrrol-2-carbonsäurepyrrolidid Hydrochlorid

27,1 g N-tert.-Butoxycarbonyl-(2S,3aS,6aS)-octahydrocyclopenta[b]-pyrrol-2-carbonsäure-pyrrolidid werden in 250 ml mit HCl gesättigtem Dimethoxyethan 2 Stunden bei Raumtemperatur gerührt. Nach Einengen erhält man 22,1 g der Titelverbindung
$R_f$ ($SiO_2$, $CH_2Cl_2$/MeOH = 4:1): 0,25;
MS (DCI) = 209 (M+1).

b) N-tert.-Butoxycarbonyl-(2S,3aS,6aS)-octahydrocyclopenta-[b]pyrrol-2-carbonsäure-pyrrolidid

40,4 g N-tert.-Butoxycarbonyl-(2S,3aS,6aS)-octahydrocyclopenta[b]pyrrol-2-carbonsäure werden zusammen mit 48,7 ml Pyrrolidin und 112,7 ml N-Ethylmorpholin in 260 ml trockenem DMF mit 108,3 ml n-Propanphoshonsäureanhydrid (50 % in $CH_2Cl_2$) analog der oben angegebenen Vorschrift umgesetzt. Man erhält 27,1 g der Titelverbindung.
$R_f$ ($SiO_2$, Ethylacetat/MeOH = 20:1) : 0,4;
MS (FAB) : 309 (M+1).

c) N-tert.-Butoxycarbonyl-(2S,3aS,6aS)-octahydrocyclopenta[b]pyrrol-2-carbonsäure

56,5 g (0,163 mol) N-tert.-Butoxycarbonyl-(2S,3aS,6aS)-octahydrocyclopenta[b]pyrrol-2-carbonsäurebenzylester werden in 400 ml Ethanol mit 2 g Palladium auf Kohle als Katalysator bei 1,1 bar und 20° C hydriert. Man erhält 40,4 g der Titelverbindung.
$R_f$ ($SiO_2$, EtOAc/MeOH = 20:1) 0,45.

d) N-tert.-Butoxycarbonyl-(2S,3aS,6aS)-octahydrocyclopenta[b]pyrrol-2-carbonsäure-benzylester

45 g (2S,3aS,6aS)-Octahydrocyclopenta[b]pyrrol-2-carbonsäurebenzylester•Hydrochlorid werden in 200 ml Dioxan/Wasser (1:1) und 300 ml 2N NaOH bei 0° C mit 45 g Di-tert.-butylpyrocarbonat ($Boc_2O$) versetzt. Nach 16 Stunden wird das Dioxan im Vakuum entfernt. Die Lösung wird mit 5N HCl sauer gestellt und mit Essigester extrahiert. Die organische Phase wird mit gesättigter NaCl-Lösung gewaschen und mit $MgSO_4$ getrocknet.
$R_f$ ($SiO_2$, EtOAc/Cyclohexan = 1:1) = 0,4.

Analog den in Beispiel 121 angegebenen Vorschriften wurden unter Verwendung geeigneter Ausgangsmaterialien hergestellt:

**Beispiel 122**

N-(3-Phenyl-propionyl)-(2S,3aS,6aS)-octahydrocyclopenta[b]pyrrol-2-carbonsäure-pyrrolidid
Schmp. 144° C; $[\alpha]_D^{20}$ = + 32,8° (c = 1, $CH_2Cl_2$).

**Beispiel 123**

N-(5-Phenyl-pentanoyl)-(2S,3aS,6aS)-octahydrocyclopenta[b]pyrrol-2-carbonsäure-pyrrolidid
Öl; $[\alpha]_D^{20}$ = + 36° (c = 1, $CH_2Cl_2$).

**Beispiel 124**

N-(4-Benzoyl-butyryl)-(2S,3aS,6aS)-octahydrocyclopenta[b]pyrrol-2-carbonsäure-pyrrolidid
Öl; $[\alpha]_D^{20}$ = + 32° (c = 1, $CH_2Cl_2$).

**Beispiel 125**

N-(3-Benzoyl-propionyl)-(2S,3aS,6aS)-octahydrocyclopenta[b]pyrrol-2-carbonsäure-pyrrolidid
Schmp. 98° C; $[\alpha]_D^{20}$ = + 40° (c = 1, $CH_2Cl_2$).

**Beispiel 126**

N-(3,4,5-Trimethoxycinnamoyl)-(2S,3aS,6aS)-octahydrocyclopenta[b]pyrrol-2-carbonsäure-pyrrolidid
Öl; $[\alpha]_D^{20}$ = + 95,7° (c = 0,49, $CH_2Cl_2$).

**Beispiel 127**

N-(4-Cyclohexyl-butyryl)-(2S,3aS,6aS)-octahydrocyclopenta[b]pyrrol-2-carbonsäure-pyrrolidid
Öl; $[\alpha]_D^{20}$ = + 36,3° (c = 0,46, $CH_2Cl_2$).

**Beispiel 128**

N-(trans-2-Phenyl-1-cyclopropyl-carbonyl)-(2S,3aS,6aS)-octahydrocyclopenta[b]pyrrol-2-carbonsäure-pyrrolidid
Öl; $[\alpha]_D^{20}$ = + 52,7° (c = 0,62, $CH_2Cl_2$).

**Beispiel 129**

N-[4-(2-Allyl-phenoxy)-butyryl]-(2S,3aS,6aS)-octahydrocyclopenta[b]pyrrol-2-carbonsäure-pyrrolidid
Öl; $[\alpha]_D^{20}$ = + 25,4° (c = 0,548, $CH_2Cl_2$).

**Beispiel 130**

N-[4-(4-tert.-Butyl-phenoxy)-butyryl]-(2S,3aS,6aS)-octahydrocyclopenta[b]pyrrol-2-carbonsäure-pyrrolidid
Öl; $[\alpha]_D^{20}$ = + 26,9 (c = 0,478, $CH_2Cl_2$).

**Beispiel 131**

N-[4-(2-Benzoyl-phenoxy)-butyryl]-(2S,3aS,6aS)-octahydrocyclopenta[b]pyrrol-2-carbonsäure-pyrrolidid
Öl; $[\alpha]_D^{20}$ = + 30° (c = 0,614, $CH_2Cl_2$).

**Beispiel 132**

N-[4-(4-Phenoxy-phenoxy)-butyryl]-(2S,3aS,6aS)-octahydrocyclopenta[b]pyrrol-2-carbonsäure-pyrrolidid
Öl; $[\alpha]_D^{20} = + 20{,}5^\circ$ (c = 0,56, $CH_2Cl_2$).

**Beispiel 133**

N-Oleoyl-(2S,3aS, 6aS)-octahydrocyclopenta[b]pyrrol-2-carbonsäure-pyrrolidid
Öl; $[\alpha]_D^{20} = + 30{,}1^\circ$ (c = 0,426, $CH_2Cl_2$).

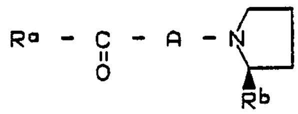

| Bsp.-Nr. | $R^a$ | A | $R^b$ |
|---|---|---|---|
| 134 | 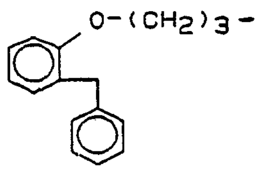 | 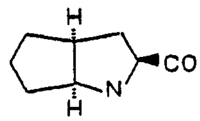 | H |
| 135 | 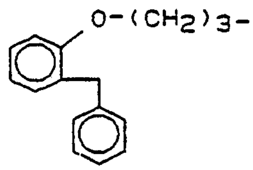 | 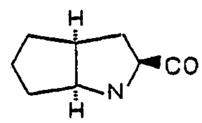 | CHO |
| 136 | $4-CH_3O-C_6H_4-(CH_2)_3-$ | 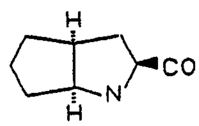 | $CO-CF_2-CONHCH_2C_6H_5$ |
| 137 | $4-CH_3O-C_6H_4-(CH_2)_3-$ | 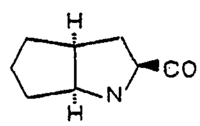 | $COCF_2CONHCHC_6H_5$<br>$CH_3$ |
| 138 | 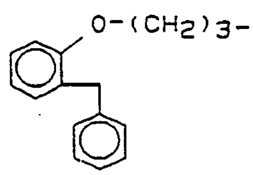 | 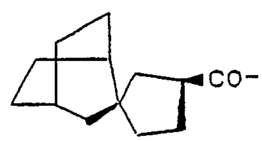 | H |
| 139 | 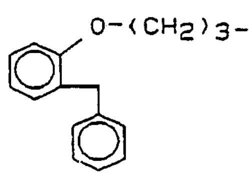 | 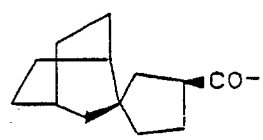 | CHO |

**Ansprüche**

1. Verbindung der Formel I

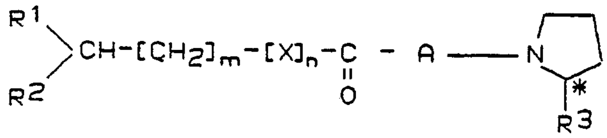

(I)

in welcher
$R^1$ Wasserstoff; $(C_1-C_{20})$-Alkyl; $(C_3-C_{20})$-Alkenyl; $(C_6-C_{12})$-Aryl, das gegebenenfalls durch einen, zwei oder drei gleiche oder verschiedene Reste aus der Reihe $(C_1-C_4)$-Alkyl, $(C_1-C_4)$-Alkoxy, Halogen, Nitro, Hydroxy, Amino, $(C_1-C_4)$-Alkylamino und Di-$(C_1-C_4)$-alkylamino, oder ein ($C_1$ oder $C_2$)-Alkylendioxy substituiert ist, bedeutet;
$R^2$ Wasserstoff; $(C_1-C_6)$-Alkyl; $(C_6-C_{12})$-Aryl; $(C_6-C_{12})$-Aryloxy; $(C_7-C_{13})$-Aroyl; Hydroxy oder $(C_1-C_4)$-Alkoxy bedeutet, wobei Aryl, Aryloxy und Aroyl jeweils gegebenenfalls durch einen, zwei oder drei gleiche oder verschiedene Reste aus der Reihe $(C_1-C_4)$-Alkyl, Phenyl-$(C_1-C_3)$-alkyl, $(C_1-C_4)$-Alkenyl, Phenyl-$(C_2-C_4)$-alkenyl, $(C_1-C_4)$-Alkoxy, $(C_1-C_4)$-Alkenyloxy, Phenyl-$(C_1-C_3)$-alkoxy, Halogen, Nitro, Hydroxy, Amino, $(C_1-C_4)$-Alkylamino und Di-$(C_1-C_4)$-alkylamino, oder ein ($C_1$ oder $C_2$)-Alkylendioxy substituiert sind, oder
$R^1$ und $R^2$ zusammen für Benzyliden stehen, worin der Phenylring gegebenenfalls durch einen, zwei oder drei gleiche oder verschiedene Reste aus der Reihe $(C_1-C_4)$-Alkyl, $(C_1-C_4)$-Alkoxy, Halogen, Nitro, Hydroxy, Amino, $(C_1-C_4)$-Alkylamino und Di-$(C_1-C_4)$-alkylamino, oder ein ($C_1$ oder $C_2$)-Alkylendioxy substituiert ist, oder $R^1$ und $R^2$ zusammen mit dem sie tragenden C-Atom für $(C_3-C_6)$- Cycloalkyl stehen, das gegebenenfalls durch Phenyl substituiert ist, bedeuten;
A einen Rest aus der Gruppe

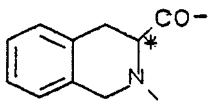 ; 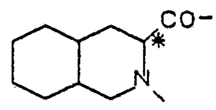 ; 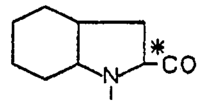 ; 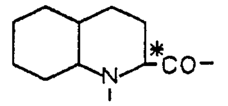 ;

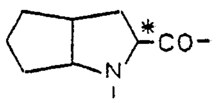 ; 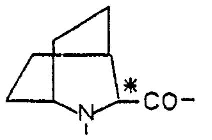 ; 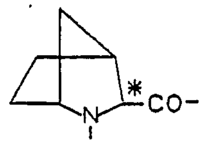 ; 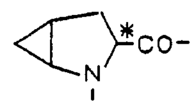 ;

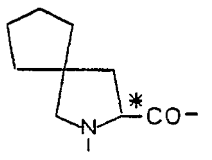 ; 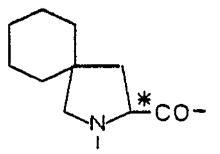 ; 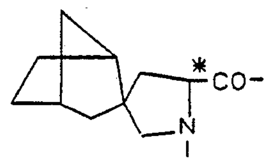 ;

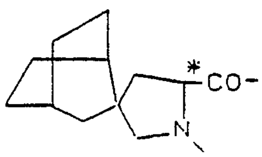 ; 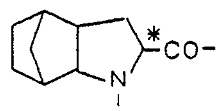 ; 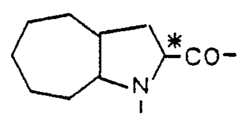 ;

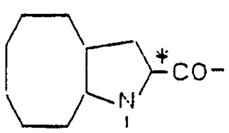 ; 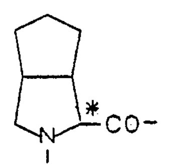 ; 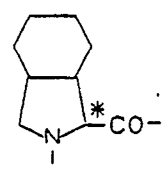 ;

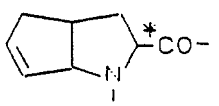 ; 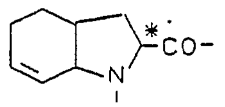 ; 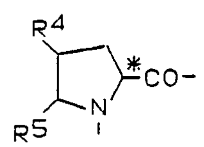 ;

bedeutet,
wobei $R^4$ und $R^5$ gleich oder verschieden sind und unabhängig voneinander Wasserstoff; $(C_1-C_6)$-Alkyl; $(C_5-C_8)$-Cycloalkyl; Phenyl oder Phenyl-$(C_1-C_4)$-alkyl, die jeweils gegebenenfalls durch $(C_1-C_4)$-Alkyl, $(C_1-C_4)$-Alkoxy oder Halogen mono- der disubstitüiert sein können, wobei jedoch höchstens einer der Reste $R^4$ oder $R^5$ Wasserstoff bedeuten kann; und
$R^3$ einen Rest aus der Gruppe Wasserstoff; Hydroxymethyl; Formyl;

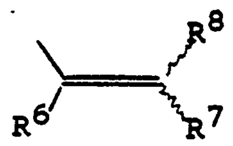 ; 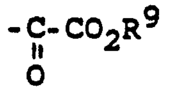 ; 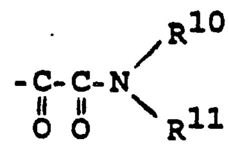 ;

$-CO-CF_3$; $-CO-CF_2-CO_2R^9$; $-CO-CF_2-R^{10}$ und

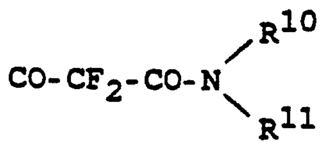 bedeuten, wobei

$R^6$ Wasserstoff; $(C_1-C_6)$-Alkyl oder $(C_6-C_{12})$-Aryl, das gegebenenfalls durch einen oder zwei gleiche oder verschiedene Reste aus der Reihe $(C_1-C_4)$-Alkyl, ($C_1$ oder $C_2$)-Alkoxy und Halogen, oder ein ($C_1$ oder $C_2$)-Alkylendioxy substituiert ist, bedeutet;
$R^7$ Wasserstoff; $(C_1-C_6)$-Alkyl; Cyano; $(C_7-C_{13})$-Aroyl, das gegebenenfalls durch einen oder zwei gleiche oder verschiedene Reste aus der Reihe $(C_1-C_4)$-Alkyl, ($C_1$ oder $C_2$)-Alkoxy, Halogen und Nitro, oder ein ($C_1$ oder $C_2$)-Alkylendioxy monosubstituiert ist; $(C_1-C_6)$-Alkoxycarbonyl oder $(C_1-C_8)$-Alkanoyl bedeutet;
$R^8$ Cyano; $(C_7-C_{13})$-Aroyl oder $(C_6-C_{12})$-Aryl-$(C_2-C_4)$-alkanoyl bedeutet, wobei jeweils Aryl durch einen, zwei oder drei gleiche oder verschiedene Reste aus der Reihe $(C_1-C_4)$-Alkyl, $(C_1-C_4)$-Alkoxy, Halogen, Nitro und Hydroxy oder ein ($C_1$ oder $C_2$)-Alkylendioxy substituiert sein kann; oder $(C_1-C_6)$-Alkanoyl; $(C_1-C_6)$-Alkoxycarbonyl; Benzyloxycarbonyl oder einen Rest der Formel

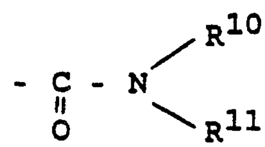

bedeutet;
$R^9$ Wasserstoff; $(C_1-C_6)$-Alkyl; Phenyl-$(C_1-C_4)$-alkyl oder Diphenyl-$(C_1-C_4)$-alkyl bedeutet;
$R^{10}$ und $R^{11}$ gleich oder verschieden sind und Wasserstoff; $(C_1-C_8)$-Alky; $(C_6-C_{12})$-Aryl oder $(C_6-C_{12})$-Aryl-$(C_1-C_8)$-alkyl, worin jeweils Aryl gegebenenfalls durch einen, zwei oder drei gleiche oder verschiedene Reste aus der Reihe $(C_1-C_4)$-Alkyl, $(C_1-C_4)$-Alkoxy, Halogen und Hydroxy oder ein ($C_1$ oder $C_2$)-Alkylendioxy substituiert ist; oder $(C_5-C_9)$-Cycloalkyl bedeuten; oder
$R^{10}$ und $R^{11}$ zusammen mit dem diese tragenden N-Atom einen 5- bis 10-gliedrigen heterocyclischen Ring bilden, der als Ringatome 2-9 Kohlenstoffatome und gegebenenfalls 1 oder 2 weitere gleiche oder verschiedene Heteroatome aus der Reihe Sauerstoff, Schwefel und Stickstoff enthält und der gegebenenfalls durch einen Rest aus der Reihe $(C_1-C_8)$-Alkyl, Phenyl, Phenyl-$(C_1-C_4)$-alkyl, Benzoyl, Phenyl-$(C_2-C_4)$-alkanoyl, 2- oder 3-Furoyl, 2-, 3- oder 4-Pyridyl und 2-, 4- oder 5-Pyrimidinyl substituiert ist, worin Phenyl, Phenylalkyl, Benzoyl und Phenylalkanoyl ihrerseits jeweils im Phenylrest durch einen, zwei oder drei gleiche oder verschiedene Reste aus der Reihe $(C_1-C_4)$-Alkyl, $(C_1-C_4)$-Alkoxy, Halogen, Hydroxy, Cyano und Nitro, oder ein ($C_1$ oder $C_2$)-Alkylendioxy substituiert sein können;
X Sauerstoff, Imino oder N-$(C_1-C_8)$-Alkylimino bedeutet;
m = 0, 1, 2, 3, 4 oder 5 ist und
n = 0 oder 1 ist
sowie deren physiologisch verträgliche Salze.

2. Verbindung der Formel I gemäß Anspruch 1, in welcher,
$R^1$ Wasserstoff; $(C_1-C_{18})$-Alkyl; $(C_3-C_{18})$-Alkenyl oder Phenyl, das gegebenenfalls durch einen oder zwei gleiche oder verschiedene Reste aus der Reihe Methyl, Ethyl, Methoxy, Fluor, Chlor, Brom, Nitro, Hydroxy,

Amino, Methylamino und Dimethylamino, oder drei Methoxy, oder ein Methylendioxy substituiert ist, bedeutet;

$R^2$ Wasserstoff; Phenyl; Phenoxy; Benzoyl; Hydroxy oder Methoxy bedeutet, wobei Phenyl, Phenoxy und Benzoyl jeweils gegebenenfalls durch einen oder zwei gleiche oder verschiedene Reste aus der Reihe Methyl, Ethyl, Benzyl, Phenethyl, Styryl, Fluor, Chlor, Brom, Nitro, Hydroxy, Amino, Methylamino und Dimethylamino, oder ein Methylendioxy substituiert sind; oder

$R^1$ und $R^2$ zusammen für Benzyliden stehen, worin der Phenylrest gegebenenfalls durch einen oder zwei gleiche oder verschiedene Reste aus der Reihe Methyl, Ethyl, Methoxy, Fluor, Chlor, Brom und Hydroxy, oder Methoxy, oder ein Methylendioxy substituiert ist, oder $R^1$ und $R^2$ mit dem sie tragenden C-Atom für Cyclopropyl stehen, das gegebenenfalls durch Phenyl substituiert ist;

$R^6$ Wasserstoff; $(C_1\text{-}C_4)$-Alkyl; Phenyl; o-, m- oder p-Methoxyphenyl; 2,3-, 2,4-, 2,5-, 2,6-, 3,4- oder 3,5-Dimethoxyphenyl; o-, m- oder p-Tolyl; 2,3-, 2,4-, 2,5-, 2,6-, 3,4- oder 3,5-Xylyl; o-, m- oder p-Fluorphenyl; o-, m- oder p-Chlorphenyl oder 2,3- oder 3,4-Methylendioxyphenyl bedeutet;

$R^7$ Wasserstoff; $(C_1\text{-}C_4)$-Alkyl; Cyano; Benzoyl, das gegebenenfalls durch einen oder zwei gleiche oder verschiedene Reste aus der Reihe Methyl, Methoxy, Fluor, Chlor, Brom und Nitro, oder ein Methylendioxy substituiert ist; $(C_1\text{-}C_4)$-Alkoxycarbonyl oder $(C_1\text{-}C_5)$-Alkanoyl bedeutet;

$R^8$ Cyano; Benzoyl, das gegebenenfalls durch einen oder zwei gleiche oder verschiedene Reste aus der Reihe Methyl, Methoxy, Fluor, Chlor und Brom, drei Methoxy, oder ein Methylendioxy substituiert ist; $(C_1\text{-}C_4)$-Alkanoyl; $(C_1\text{-}C_4)$-Alkoxycarbonyl oder einen Rest der Formel

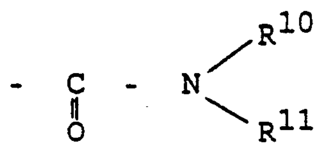

bedeutet;

$R^9$ $(C_1\text{-}C_4)$-Alkyl; Benzyl; Phenethyl oder Benzhydryl bedeutet;

$R^{10}$ und $R^{11}$ gleich oder verschieden sind und Wasserstoff; $(C_1\text{-}C_6)$-Alkyl; Phenyl oder Phenyl-$(C_1\text{-}C_4)$-alkyl, worin der Phenylrest jeweils durch einen oder zwei gleiche oder verschiedene Reste aus der Reihe Methyl, Ethyl, Methoxy, Fluor, Chlor, Brom und Hydroxy, drei Methoxy oder ein Methylendioxy substituiert sein kann; Cyclopentyl; Cyclohexyl oder Cycloheptyl bedeuten; oder $R^{10}$ und $R^{11}$ zusammen mit dem diese tragenden N-Atom einen 5- bis 7-gliedrigen heterocyclischen Ring bilden, der als Ringatome 2-6 Kohlenstoffatome und gegebenenfalls 1 oder 2 weitere gleiche oder verschiedene Heteroatome aus der Reihe Sauerstoff, Schwefel und Stickstoff enthält und der gegebenenfalls durch einen Rest aus der Reihe $(C_1\text{-}C_4)$-Alkyl, Phenyl, Phenyl-$(C_1\text{-}C_4)$-alkyl, Benzoyl, 2- oder 3-Furoyl, 2-, 3-oder 4-Pyridyl und 2-, 4- oder 5-Pyrimidinyl substituiert ist, worin Phenyl, Phenylalkyl und Benzoyl ihrerseits jeweils im Phenylrest durch einen oder zwei gleiche oder verschiedene Reste aus der Reihe Methyl, Methoxy, Fluor, Chlor, Brom, Cyano, Nitro und Hydroxy, drei Methoxy oder ein Methylendioxy substituiert sein können;

X Sauerstoff bedeutet;

m = 0, 1, 2, 3, 4 oder 5 ist;

n = 0 oder 1 ist,

sowie deren physiologisch verträgliche Salze.

3. Verbindung der Formel I gemäß einem oder mehreren der Ansprüche 1 bis 2, in welcher

$R^1$ Wasserstoff; Methyl; Ethyl; Propyl; Isopropyl; Phenyl; o-, m- oder p-Tolyl; o-, m- oder p-Chlorphenyl; o-, m-oder p-Fluorphenyl; o-, m- oder p-Methoxyphenyl oder 2,3-, 2,4-, 2,5-, 2,6-, 3,4- oder 3,5-Dimethoxyphenyl bedeutet;

$R^2$ Wasserstoff; Phenyl; o-, m- oder p-Tolyl; o-, m- oder p-Chlorphenyl; o-, m- oder p-Fluorphenyl; o-, m- oder p-Methoxyphenyl; 2,3-, 2,4-, 2,5-, 2,6-, 3,4 oder 3,5-Dimethoxyphenyl; Phenoxy; o-, m- oder p-Tolyloxy; o-, m- oder p-Chlorphenoxy; o-, m- oder p-Fluorphenoxy; o-, m- oder p-Methoxyphenoxy; 2,3-, 2,4-, 2,5-, 2,6-, 3,4- oder 3,5-Dimethoxyphenoxy; 2-, 3- oder 4-Benzylphenoxy; 2-, 3- oder 4-Phenethyloxy; 2-, 3- oder 4-Styryloxy; Benzoyl; o-, m- oder p-Toluoyl; o-, m- oder p-Chlorbenzoyl; o-, m- oder p-Fluorbenzoyl; o-, m- oder p-Methoxybenzoyl; 2,3-, 2,4-, 2,5-, 2,6-, 3,4- oder 3,5-Dimethoxybenzoyl; Hydroxy oder Methoxy bedeutet;

$R^1$ und $R^2$ zusammen für Benzyliden; o-, m- oder p-Methylbenzyliden; o-, m- oder p-Methoxybenzyliden, 2,3-, 2,4-, 2,5-, 2,6-, 3,4- oder 3,5-Dimethoxybenzyliden oder 2-Phenyl-1-cyclopropyl stehen;

$R^6$ Wasserstoff oder Methyl bedeutet;

$R^7$ Wasserstoff; Methyl; Cyano; Benzoyl; o-, m- oder p-Methoxybenzoyl; o-, m- oder p-Chlorbenzoyl; 2,3-, 2,4-, 2,5-, 2,6-, 3,4- oder 3,5-Dimethoxybenzoyl; Acetyl; Methoxycarbonyl oder Ethoxycarbonyl bedeutet;
$R^8$ Cyano; Formyl; Benzoyl; o-, m- oder p-Methoxybenzoyl; o-, m- oder p-Chlorbenzoyl, 2,3-, 2,4-, 2,5-, 2,6-, 3,4-oder 3,5-Dimethoxybenzoyl; Acetyl; Methoxycarbonyl; Ethoxycarbonyl oder einen Rest der Formel

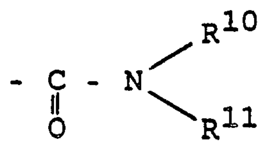

bedeutet;
$R^9$ Methyl; Ethyl; Benzyl oder Benzhydryl bedeutet;
$R^{10}$ und $R^{11}$ gleich oder verschieden sind und Wasserstoff; $(C_1-C_6)$-Alkyl; Phenyl oder Phenyl-$(C_1-C_4)$-alkyl, worin der Phenylrest jeweils durch einen oder zwei gleiche oder verschiedene Reste aus der Reihe Methyl, Methoxy, Fluor und Chlor, drei Methoxy, oder ein Methylendioxy substituiert sein kann; Cyclopentyl oder Cyclohexyl bedeutet; oder
$R^{10}$ und $R^{11}$ zusammen mit dem diese tragenden N-Atom einen 5-bis 7-gliedrigen heterocyclischen Ring bilden, der als Ringatome 2-6 Kohlenstoffatome und gegebenenfalls 1 oder 2 weitere gleiche oder verschiedene Heteroatome aus der Reihe Sauerstoff, Schwefel und Stickstoff enthält und der gegebenenfalls durch Methyl, Phenyl, Phenyl-$(C_1-C_4)$-alkyl, Benzoyl, Pyridyl oder Pyrimidinyl monosubstituiert ist, worin Phenyl, Phenylalkyl und Benzoyl ihrerseits jeweils im Phenylrest durch ein oder zwei gleiche oder verschiedene Reste aus der Reihe Methyl, Methoxy, Fluor, Chlor, Cyano und Nitro, oder drei Methoxy substituiert sein können;
X Sauerstoff bedeutet;
m = 0, 1, 2, 3, 4 oder 5 ist und
n = 0 oder 1 ist,
sowie deren physiologisch verträgliche Salze.

4. Verbindung der Formel I gemäß einem oder mehreren der Ansprüche 1 bis 3, in welcher
$R^2$ Wasserstoff bedeutet;
$R^6$ Wasserstoff bedeutet;
$R^8$ Wasserstoff bedeutet;
X Sauerstoff bedeutet;
m = 1, 2, 3 oder 4 ist und
n = 0 oder 1 ist.
sowie deren physiologisch verträglichen Salze.

5. Verfahren zur Herstellung einer Verbindung der Formel I gemäß einem oder mehreren der Ansprüche 1 bis 4, dadurch gekennzeichnet, daß man
a) eine Verbindung der Formel II

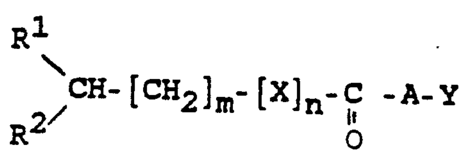

(II)

in welcher $R^1$, $R^2$, A, X, m und n die gleiche Bedeutung wie in Formel I haben und Y Hydroxy, $(C_1-C_{10})$-Alkoxy, $(C_6-C_{12})$-Aryloxy, $(C_6-C_{12})$-Aryl-$(C_1-C_6)$-alkoxy oder den Rest eines aktivierten Säurederivats bedeuten, mit einer Verbindung der Formel III umsetzt,

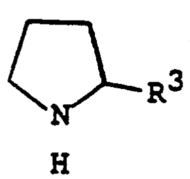

(III)

in welcher $R^3$ die gleiche Bedeutung wie in Formel I hat; oder

b) für den Fall, daß $R^3$ Hydroxymethyl oder Formyl bedeutet, eine Verbindung der Formel II mit der Verbindung der Formel IV umsetzt,

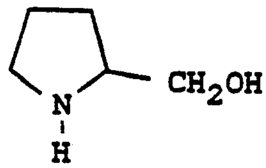

(IV)

und gegebenenfalls anschließend die Alkoholfunktion zum Aldehyd oxidiert; oder

$c_1$) für den Fall, daß $R^3$ den Rest

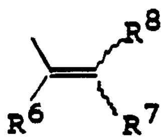

bedeutet, eine Verbindung der Formel V,

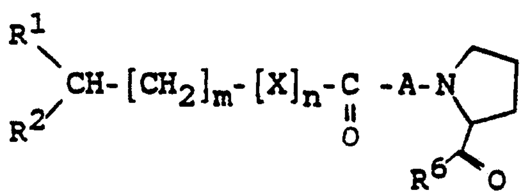

(V)

in welcher $R^1$, $R^2$, $R^6$, A, X, m und n die gleiche Bedeutung wie in Formel I haben, in Gegenwart einer Base umsetzt mit einer Verbindung der Formel VI

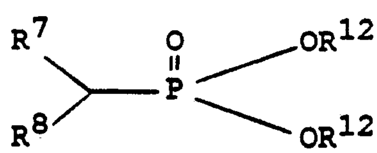

(VI)

in welcher $R^7$ und $R^8$ die gleiche Bedeutung wie in Formel I haben und $R^{12}$ für $(C_1$-$C_4)$-Alkyl, Phenyl oder Phenyl-$(C_1$-$C_4)$-alkyl steht; oder

$c_2$) eine Verbindung der unter ($c_1$) definierten Formel V umsetzt mit einer Verbindung der Formel VII

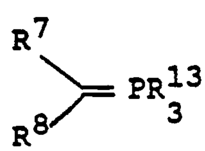

(VII)

in welcher $R^7$ und $R^8$ die gleiche Bedeutung wie in Formel I haben und $R^{13}$ für Phenyl, $(C_1$-$C_6)$-Alkylphenyl oder Di-$(C_1$-$C_6)$-alkylphenyl steht; oder

$c_3$) zur Herstellung einer Verbindung der Formel I, worin $R^8$ für einen Rest der Formel

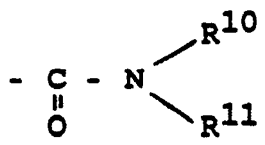

steht,
eine Verbindung der Formel I, in welcher $R^8$ ($C_1$-$C_6$)-Alkoxycarbonyl bedeutet und die übrigen Reste und Variablen wie oben definiert sind, umsetzt mit einer Verbindung der Formel VIII

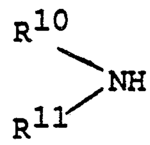 (VIII)

in welcher $R^{10}$ und $R^{11}$ die gleiche Bedeutung wie in Formel I haben; oder

$c_4$) eine Verbindung der Formel I, in welcher $R^8$ Carboxy bedeutet und die übrigen Reste und Variablen wie oben definiert sind, nach deren Umwandlung in ein aktiviertes Derivat mit einer Verbindung der unter ($c_3$) definierten Formel VIII umsetzt; oder

d) für den Fall, daß $R^3$ den Rest -CO-$CF_3$, -CO-$CF_2$-$R^{10}$ oder -CO-$CF_2$-$CO_2R^9$ bedeutet, eine Verbindung der Formel I, in welcher $R^3$ Formyl bedeutet, mit einer Verbindung der Formel IX umsetzt,
Z-$CF_2$-$R^{14}$ (IX)
in welcher $R^{14}$ die Bedeutung von $R^{10}$ hat oder für Fluor oder -$CO_2R^9$ steht und Z Halogen, insbesondere Brom oder Jod bedeutet, und das erhaltene Produkt oxidiert; oder

$d_1$) für den Fall, daß $R^3$ den Rest -CO-$CF_2$-$R^{10}$ darstellt, eine Verbindung der Formel I, in welcher $R^3$ für -CO-$CF_2$-$CO_2R^9$ steht, wobei $R^9$ Wasserstoff bedeutet, zunächst mit einer Verbindung der Formel X umsetzt,

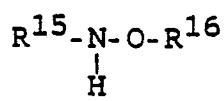 (X)

in welcher $R^{15}$ und $R^{16}$ gleich oder verschieden und unabhängig voneinander ($C_1$-$C_4$)-Alkyl bedeuten können, oder mit den sie tragenden Heteroatomen einen 5-7-gliedrigen Ring bilden, das Produkt anschließend mit einer Verbindung der Formel XI umsetzt,
$R^{10}$-Mg-W (XI)
in welcher $R^{10}$ die gleiche Bedeutung wie in Formel I hat, und W für Halogen, insbesondere für Chlor, Brom oder Jod steht, und das erhaltene Produkt oxidiert; oder

$d_2$) für den Fall, daß $R^3$ für

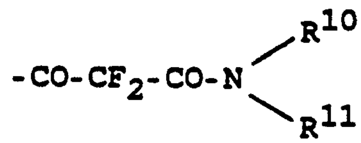

steht, eine Verbindung der Formel XII

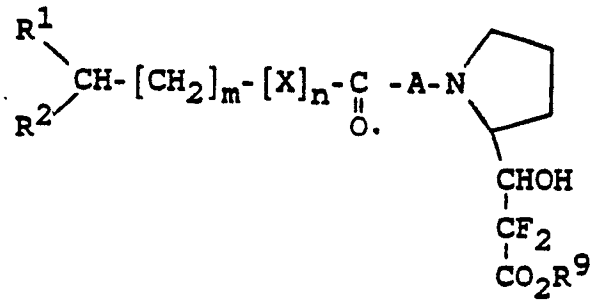

(XII)

in welcher $R^1$, $R^2$, $R^9$, A, X, m und n die gleiche Bedeutung wie in Formel I haben, mit einer Verbindung der Formel VIII umsetzt und anschließend oxidiert; oder

e) für den Fall, daß $R^3$ für $-CO-CO_2R^9$ steht, eine Verbindung der Formel XIII,

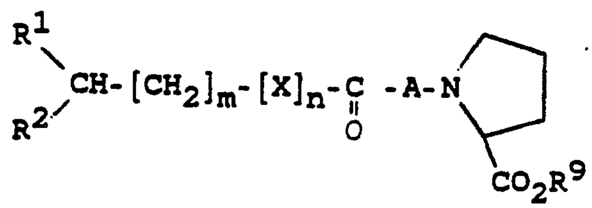

(XIII)

in welcher $R^1$, $R^2$, $R^9$, A, X, m und n die gleiche Bedeutung wie in Formel I haben, zunächst mit der Verbindung der Formel XIV

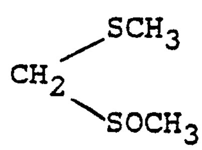

(XIV)

in Gegenwart einer Base umsetzt, und das Produkt anschließend mit einem Kupfer- oder Quecksilbersalz in einem Alkohol $R^9$-OH als Lösungsmittel zur Reaktion bringt; oder

f) für den Fall, daß $R^3$ den Rest

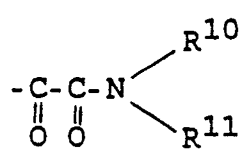

bedeutet, eine Verbindung der Formel I, in welcher $R^3$ $-CO-CO_2R^9$ bedeutet, zunächst gegebenenfalls zur freien Säure verseift und anschließend mit einer Verbindung der Formel VIII umsetzt und die nach (a)-(f) erhaltenen Verbindungen gegebenenfalls in ihre physiologisch verträglichen Salze überführt.

6. Verwendung einer Verbindung gemäß einem oder mehreren der Ansprüche 1 bis 4 bis Heilmittel.

7. Verwendung einer Verbindung gemäß einem oder mehreren der Ansprüche 1 bis 4 als Prolyl-Endopeptidase-Hemmer.

8. Pharmazeutische Zubereitung, enthaltend eine Verbindung der Formel I gemäß einem oder mehreren der Ansprüch 1 bis 4.

9. Verfahren zur Herstellung einer Zubereitung gemäß Anspruch 8, dadurch gekennzeichnet, daß man den Wirkstoff zusammen mit einem physiologisch annehmbaren Träger und gegebenenfalls weiteren Zusatz-, Hilfs- und/oder Konservierungsstoffen in eine geeignete Darreichungsform bringt.

Patentansprüche für folgende Vertragsstaaten: ES, GR

1. Verfahren zur Herstellung einer Verbindung der Formel I

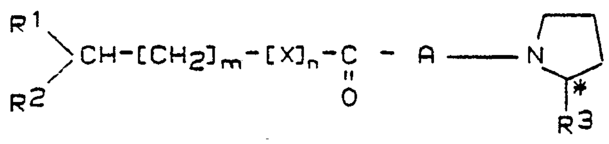

(I)

in welcher

$R^1$ Wasserstoff; $(C_1-C_{20})$-Alkyl; $(C_3-C_{20})$-Alkenyl; $(C_6-C_{12})$-Aryl, das gegebenenfalls durch einen, zwei oder drei gleiche oder verschiedene Reste aus der Reihe $(C_1-C_4)$-Alkyl, $(C_1-C_4)$-Alkoxy, Halogen, Nitro, Hydroxy, Amino, $(C_1-C_4)$-Alkylamino und Di-$(C_1-C_4)$-alkylamino, oder ein ($C_1$ oder $C_2$)-Alkylendioxy substituiert ist, bedeutet;

$R^2$ Wasserstoff; $(C_1-C_6)$-Alkyl; $(C_6-C_{12})$-Aryl; $(C_6-C_{12})$-Aryloxy; $(C_7-C_{13})$-Aroyl; Hydroxy oder $(C_1-C_4)$-Alkoxy bedeutet, wobei Aryl, Aryloxy und Aroyl jeweils gegebenenfalls durch einen, zwei oder drei gleiche oder verschiedene Reste aus der Reihe $(C_1-C_4)$-Alkyl, Phenyl-$(C_1-C_3)$-alkyl, $(C_1-C_4)$-Alkenyl, Phenyl-$(C_2-C_4)$-alkenyl, $(C_1-C_4)$-Alkoxy, $(C_1-C_4)$-Alkenyloxy, Phenyl-$(C_1-C_3)$-alkoxy, Halogen, Nitro, Hydroxy, Amino, $(C_1-C_4)$-Alkylamino und Di-$(C_1-C_4)$-alkylamino, oder ein ($C_1$ oder $C_2$)-Alkylendioxy substituiert sind, oder

$R^1$ und $R^2$ zusammen für Benzyliden stehen, worin der Phenylring gegebenenfalls durch einen, zwei oder drei gleiche oder verschiedene Reste aus der Reihe $(C_1-C_4)$-Alkyl, $(C_1-C_4)$-Alkoxy, Halogen, Nitro, Hydroxy, Amino, $(C_1-C_4)$-Alkylamino und Di-$(C_1-C_4)$-alkylamino, oder ein ($C_1$ oder $C_2$)-Alkylendioxy substituiert ist, oder $R^1$ und $R^2$ zusammen mit dem sie tragenden C-Atom für $(C_3-C_6)$- Cycloalkyl stehen, das gegebenenfalls durch Phenyl substituiert ist, bedeuten;

A einen Rest aus der Gruppe

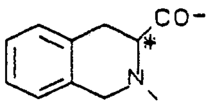 ; 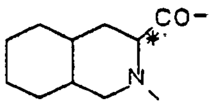 ; 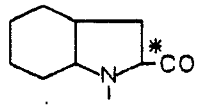 ; 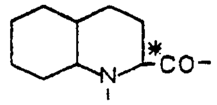 ;

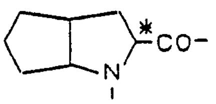 ; 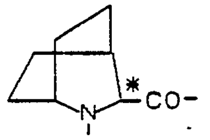 ; 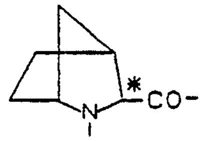 ; 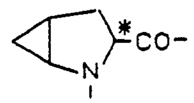 ;

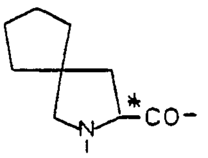 ; 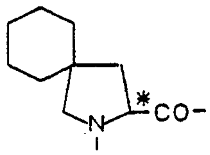 ; 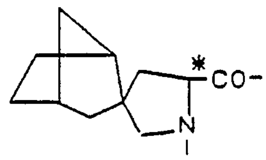 ;

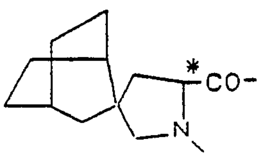 ; 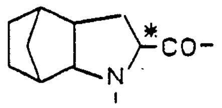 ; 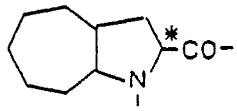 ;

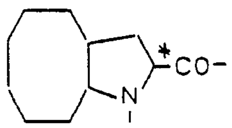 ; 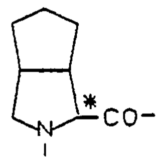 ; 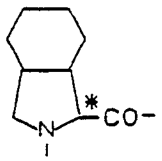 ;

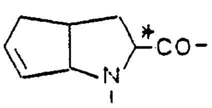 ; 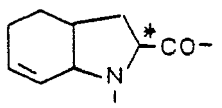 ; 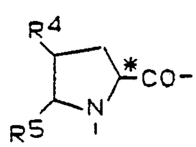 ;

bedeutet,
wobei $R^4$ und $R^5$ gleich oder verschieden sind und unabhängig voneinander Wasserstoff; $(C_1-C_6)$-Alkyl; $(C_5-C_8)$-Cycloalkyl; Phenyl oder Phenyl-$(C_1-C_4)$-alkyl, die jeweils gegebenenfalls durch $(C_1-C_4)$-Alkyl, $(C_1-C_4)$-Alkoxy oder Halogen mono- oder disubstituiert sein können, wobei jedoch höchstens einer der Reste $R^4$ oder $R^5$ Wasserstoff bedeuten kann; und
$R^3$ einen Rest aus der Gruppe Wasserstoff; Hydroxymethyl; Formyl;

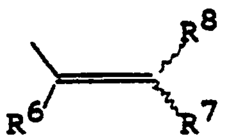 ; 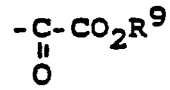 ; 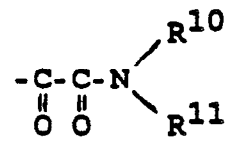 ;

$-CO-CF_3$; $-CO-CF_2-CO_2R^9$; $-CO-CF_2-R^{10}$ und

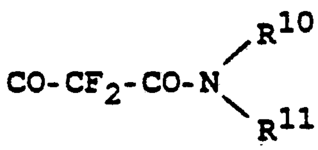 bedeuten, wobei

$R^6$ Wasserstoff; ($C_1$-$C_6$)-Alkyl oder ($C_6$-$C_{12}$)-Aryl, das gegebenenfalls durch einen oder zwei gleiche oder verschiedene Reste aus der Reihe ($C_1$-$C_4$)-Alkyl, ($C_1$ oder $C_2$)-Alkoxy und Halogen, oder ein ($C_1$ oder $C_2$)-Alkylendioxy substituiert ist, bedeutet;
$R^7$ Wasserstoff; ($C_1$-$C_6$)-Alkyl; Cyano; ($C_7$-$C_{13}$)-Aroyl, das gegebenenfalls durch einen oder zwei gleiche oder verschiedene Reste aus der Reihe ($C_1$-$C_4$)-Alkyl, ($C_1$ oder $C_2$)-Alkoxy, Halogen und Nitro, oder ein ($C_1$ oder $C_2$)-Alkylendioxy monosubstituiert ist; ($C_1$-$C_6$)-Alkoxycarbonyl oder ($C_1$-$C_8$)-Alkanoyl bedeutet;
$R^8$ Cyano; ($C_7$-$C_{13}$)-Aroyl oder ($C_6$-$C_{12}$)-Aryl-($C_2$-$C_4$)-alkanoyl bedeutet, wobei jeweils Aryl durch einen, zwei oder drei gleiche oder verschiedene Reste aus der Reihe ($C_1$-$C_4$)-Alkyl, ($C_1$-$C_4$)-Alkoxy, Halogen, Nitro und Hydroxy oder ein ($C_1$ oder $C_2$)-Alkylendioxy substituiert sein kann; oder ($C_1$-$C_6$)-Alkanoyl; ($C_1$-$C_6$)-Alkoxycarbonyl; Benzyloxycarbonyl oder einen Rest der Formel

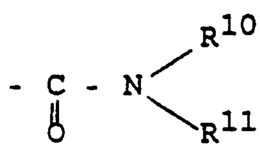

bedeutet;
$R^9$ Wasserstoff; ($C_1$-$C_6$)-Alkyl; Phenyl-($C_1$-$C_4$)-alkyl oder Diphenyl-($C_1$-$C_4$)-alkyl bedeutet;
$R^{10}$ und $R^{11}$ gleich oder verschieden sind und Wasserstoff; ($C_1$-$C_8$)-Alkyl; ($C_6$-$C_{12}$)-Aryl oder ($C_6$-$C_{12}$)-Aryl-($C_1$-$C_8$)-alkyl, worin jeweils Aryl gegebenenfalls durch einen, zwei oder drei gleiche oder verschiedene Reste aus der Reihe ($C_1$-$C_4$)-Alkyl, ($C_1$-$C_4$)-Alkoxy, Halogen und Hydroxy oder ein ($C_1$ oder $C_2$)-Alkylendioxy substituiert ist; oder ($C_5$-$C_9$)-Cycloalkyl bedeuten; oder
$R^{10}$ und $R^{11}$ zusammen mit dem diese tragenden N-Atom einen 5- bis 10-gliedrigen heterocyclischen Ring bilden, der als Ringatome 2-9 Kohlenstoffatome und gegebenenfalls 1 oder 2 weitere gleiche oder verschiedene Heteroatome aus der Reihe Sauerstoff, Schwefel und Stickstoff enthält und der gegebenenfalls durch einen Rest aus der Reihe ($C_1$-$C_8$)-Alkyl, Phenyl, Phenyl-($C_1$-$C_4$)-alkyl, Benzoyl, Phenyl-($C_2$-$C_4$)-alkanoyl, 2- oder 3-Furoyl, 2-, 3- oder 4-Pyridyl und 2-, 4- oder 5-Pyrimidinyl substituiert ist, worin Phenyl, Phenylalkyl, Benzoyl und Phenylalkanoyl ihrerseits jeweils im Phenylrest durch einen, zwei oder drei gleiche oder verschiedene Reste aus der Reihe ($C_1$-$C_4$)-Alkyl, ($C_1$-$C_4$)-Alkoxy, Halogen, Hydroxy, Cyano und Nitro, oder ein ($C_1$ oder $C_2$)-Alkylendioxy substituiert sein können;
X Sauerstoff, Imino oder N-($C_1$-$C_8$)-Alkylimino bedeutet;
m = 0, 1, 2, 3, 4 oder 5 ist und
n = 0 oder 1 ist
sowie deren physiologisch verträgliche Salze, dadurch gekennzeichnet, daß man
a) eine Verbindung der Formel II

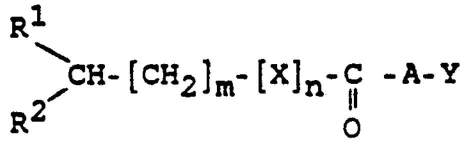

(II)

in welcher $R^1$, $R^2$, A, X, m und n die gleiche Bedeutung wie in Formel I haben und Y Hydroxy, $(C_1-C_{10})$-Alkoxy, $(C_6-C_{12})$-Aryloxy, $(C_6-C_{12})$-Aryl-$(C_1-C_6)$-alkoxy oder den Rest eines aktivierten Säurederivats bedeuten, mit einer Verbindung der Formel III umsetzt,

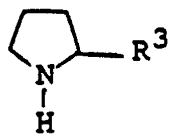

(III)

in welcher $R^3$ die gleiche Bedeutung wie in Formel I hat; oder

b) für den Fall, daß $R^3$ Hydroxymethyl oder Formyl bedeutet, eine Verbindung der Formel II mit der Verbindung der Formel IV umsetzt,

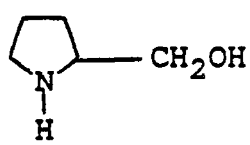

(IV)

und gegebenenfalls anschließend die Alkoholfunktion zum Aldehyd oxidiert; oder

$c_1$) für den Fall, daß $R^3$ den Rest

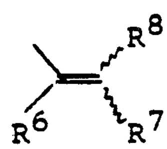

bedeutet, eine Verbindung der Formel V,

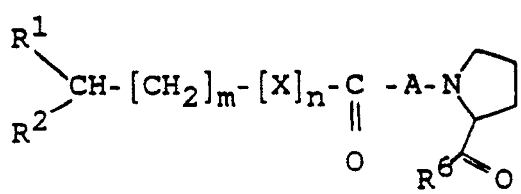

(V)

in welcher $R^1$, $R^2$, $R^6$, A, X, m und n die gleiche Bedeutung wie in Formel I haben, in Gegenwart einer Base umsetzt mit einer Verbindung der Formel VI

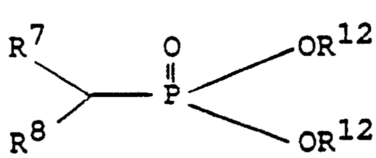

(VI)

in welcher $R^7$ und $R^8$ die gleiche Bedeutung wie in Formel I haben und $R^{12}$ für $(C_1\text{-}C_4)$-Alkyl, Phenyl oder Phenyl-$(C_1\text{-}C_4)$-alkyl steht; oder

$c_2$) eine Verbindung der unter $(c_1)$ definierten Formel V umsetzt mit einer Verbindung der Formel VII

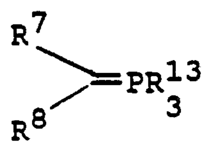

(VII)

in welcher $R^7$ und $R^8$ die gleiche Bedeutung wie in Formel I haben und $R^{13}$ für Phenyl, $(C_1\text{-}C_6)$-Alkylphenyl oder Di-$(C_1\text{-}C_6)$-alkylphenyl steht; oder

$c_3$) zur Herstellung einer Verbindung der Formel I, worin $R^8$ für einen Rest der Formel

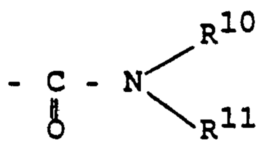

steht,

eine Verbindung der Formel I, in welcher $R^8$ $(C_1\text{-}C_6)$-Alkoxycarbonyl bedeutet und die übrigen Reste und Variablen wie oben definiert sind, umsetzt mit einer Verbindung der Formel VIII

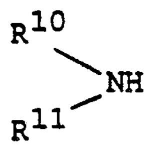

(VIII)

in welcher $R^{10}$ und $R^{11}$ die gleiche Bedeutung wie in Formel I haben; oder

$c_4$) eine Verbindung der Formel I, in welcher $R^8$ Carboxy bedeutet und die übrigen Reste und Variablen wie oben definiert sind, nach deren Umwandlung in ein aktiviertes Derivat mit einer Verbindung der unter $(c_3)$ definierten Formel VIII umsetzt; oder

d) für den Fall, daß $R^3$ den Rest $-CO-CF_3$, $-CO-CF_2-R^{10}$ oder $-CO-CF_2-CO_2R^9$ bedeutet, eine Verbindung der Formel I, in welcher $R^3$ Formyl bedeutet, mit einer Verbindung der Formel IX umsetzt,

$Z-CF_2-R^{14}$ (IX)

in welcher $R^{14}$ die Bedeutung von $R^{10}$ hat oder für Fluor oder $-CO_2R^9$ steht und Z Halogen, insbesondere Brom oder Jod bedeutet, und das erhaltene Produkt oxidert; oder

$d_1$) für den Fall, daß $R^3$ den Rest $-CO-CF_2-R^{10}$ darstellt, eine Verbindung der Formel I, in welcher $R^3$ für $-CO-CF_2-CO_2R^9$ steht, wobei $R^9$ Wasserstoff bedeutet, zunächst mit einer Verbindung der Formel X umsetzt,

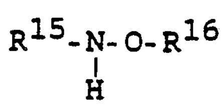

(X)

in welcher $R^{15}$ und $R^{16}$ gleich oder verschieden und unabhängig voneinander $(C_1\text{-}C_4)$-Alkyl bedeuten können, oder mit den sie tragenden Heteroatomen einen 5-7-gliedrigen Ring bilden, das Produkt anschließend mit einer Verbindung der Formel XI umsetzt,

$R^{10}-Mg-W$ (XI)

in welcher $R^{10}$ die gleiche Bedeutung wie in Formel I hat, und W für Halogen, insbesondere für Chlor, Brom oder Jod steht, und das erhaltene Produkt oxidiert; oder

$d_2$) für den Fall, daß $R^3$ für

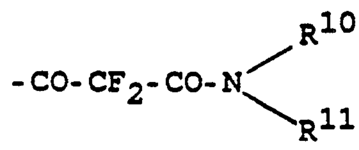

steht, eine Verbindung der Formel XII

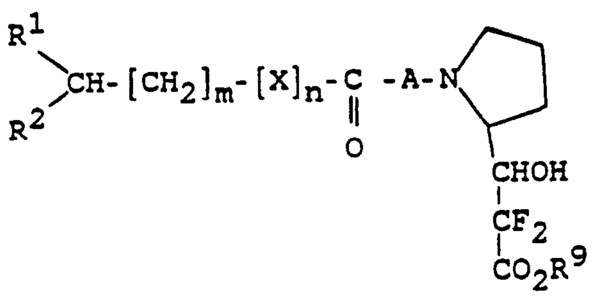

(XII)

in welcher $R^1$, $R^2$, $R^9$, A, X, m und n die gleiche Bedeutung wie in Formel I haben, mit einer Verbindung der Formel VIII umsetzt und anschließend oxidiert; oder

e) für den Fall, daß $R^3$ für $-CO-CO_2R^9$ steht, eine Verbindung der Formel XIII,

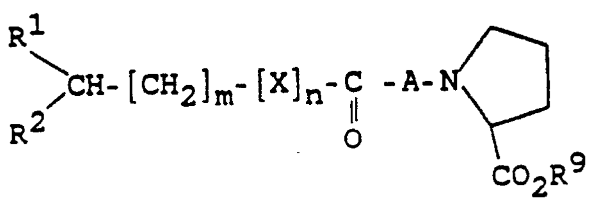

(XIII)

in welcher $R^1$, $R^2$, $R^9$, A, X, m und n die gleiche Bedeutung wie in Formel I haben, zunächst mit der Verbindung der Formel XIV

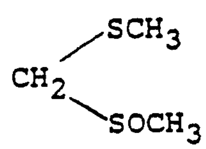

(XIV)

in Gegenwart einer Base umsetzt, und das Produkt anschließend mit einem Kupfer- oder Quecksilbersalz in einem Alkohol $R^9$-OH als Lösungsmittel zur Reaktion bringt; oder

f) für den Fall, daß $R^3$ den Rest

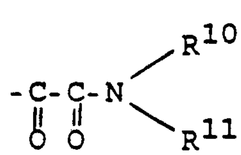

bedeutet, eine Verbindung der Formel I, in welcher $R^3$ $-CO-CO_2R^9$ bedeutet, zunächst gegebenenfalls zur freien Säure verseift und anschließend mit einer Verbindung der Formel VIII umsetzt und die nach (a)-(f) erhaltenen Verbindungen gegebenenfalls in ihre physiologisch verträglichen Salze überführt.

2. Verfahren gemäß Anspruch 1, dadurch gekennzeichnet, daß eine Verbindung der Formel I herstellt, in welcher
$R^1$ Wasserstoff; $(C_1-C_{18})$-Alkyl; $(C_3-C_{18})$-Alkenyl oder Phenyl, das gegebenenfalls durch einen oder zwei gleiche oder verschiedene Reste aus der Reihe Methyl, Ethyl, Methoxy, Fluor, Chlor, Brom, Nitro, Hydroxy, Amino, Methylamino und Dimethylamino, oder drei Methoxy, oder ein Methylendioxy substituiert ist, bedeutet;
$R^2$ Wasserstoff; Phenyl; Phenoxy; Benzoyl; Hydroxy oder Methoxy bedeutet, wobei Phenyl, Phenoxy und Benzoyl jeweils gegebenenfalls durch einen oder zwei gleiche oder verschiedene Reste aus der Reihe Methyl, Ethyl, Benzyl, Phenethyl, Styryl, Fluor, Chlor, Brom, Nitro, Hydroxy, Amino, Methylamino und Dimethylamino, oder ein Methylendioxy substituiert sind; oder
$R^1$ und $R^2$ zusammen für Benzyliden stehen, worin der Phenylrest gegebenenfalls durch einen oder zwei gleiche oder verschiedene Reste aus der Reihe Methyl, Ethyl, Methoxy, Fluor, Chlor, Brom und Hydroxy, oder Methoxy, oder ein Methylendioxy substituiert ist; oder $R^1$ und $R^2$ zusammem mit dem sie tragenden C-Atom für Cyclopropyl stehen, das gegebenenfalls durch Phenyl substituiert ist;
$R^6$ Wasserstoff; $(C_1-C_4)$-Alkyl; Phenyl; o-, m- oder p-Methoxyphenyl; 2,3-, 2,4-, 2,5-, 2,6-, 3,4- oder 3,5-Dimethoxyphenyl; o-, m- oder p-Tolyl; 2,3,-, 2,4-, 2,5-, 2,6-, 3,4- oder 3,5-Xylyl; o-, m- oder p-Fluorphenyl; o-, m- oder p-Chlorphenyl oder 2,3- oder 3,4-Methylendioxyphenyl bedeutet;
$R^7$ Wasserstoff; $(C_1-C_4)$-Alkyl; Cyano; Benzoyl, das gegebenenfalls durch einen oder zwei gleiche oder verschiedene Reste aus der Reihe Methyl, Methoxy, Fluor, Chlor, Brom und Nitro, oder ein Methylendioxy substituiert ist; $(C_1-C_4)$-Alkoxycarbonyl oder $(C_1-C_5)$-Alkanoyl bedeutet;
$R^8$ Cyano; Benzoyl, das gegebenenfalls durch einen oder zwei gleiche oder verschiedene Reste aus der Reihe Methyl, Methoxy, Fluor, Chlor und Brom, drei Methoxy, oder ein Methylendioxy substituiert ist; $(C_1-C_4)$-Alkanoyl; $(C_1-C_4)$-Alkoxycarbonyl oder einen Rest der Formel

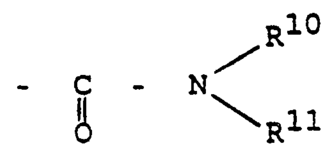

bedeutet;
$R^9$ $(C_1-C_4)$-Alkyl; Benzyl; Phenethyl oder Benzhydryl bedeutet;
$R^{10}$ und $R^{11}$ gleich oder verschieden sind und Wasserstoff; $(C_1-C_6)$-Alkyl; Phenyl oder Phenyl-$(C_1-C_4)$-alkyl, worin der Phenylrest jeweils durch einen oder zwei gleiche oder verschiedene Reste aus der Reihe Methyl, Ethyl, Methoxy, Fluor, Chlor, Brom und Hydroxy, drei Methoxy oder ein Methylendioxy substituiert sein kann; Cyclopentyl; Cyclohexyl oder Cycloheptyl bedeuten; oder $R^{10}$ und $R^{11}$ zusammen mit dem diese tragenden N-Atom einen 5- bis 7-gliedrigen heterocyclischen Ring bilden, der als Ringatome 2-6 Kohlenstoffatome und gegebenenfalls 1 oder 2 weitere gleiche oder verschiedene Heteroatome aus der Reihe Sauerstoff, Schwefel und Stickstoff enthält und der gegebenenfalls durch einen Rest aus der Reihe $(C_1-C_4)$-Alkyl, Phenyl, Phenyl-$(C_1-C_4)$-alkyl, Benzoyl, 2- oder 3-Furoyl, 2-, 3-oder 4-Pyridyl und 2-, 4- oder 5-Pyrimidinyl substituiert ist, worin Phenyl, Phenylalkyl und Benzoyl ihrerseits jeweils im Phenylrest durch einen oder zwei gleiche oder verschiedene Reste aus der Reihe Methyl, Methoxy, Fluor, Chlor, Brom, Cyano, Nitro und Hydroxy, drei Methoxy oder ein Methylendioxy substituiert sein können;
X Sauerstoff bedeutet;
m = 0, 1, 2, 3, 4 oder 5 ist;
n = 0 oder 1 ist,
sowie deren physiologisch verträgliche Salze.

3. Verfahren gemäß einem oder mehreren der Ansprüche 1 bis 2, dadurch gekennzeichnet, daß man eine Verbindung der Formel I herstellt, in welcher
$R^1$ Wasserstoff; Methyl; Ethyl; Propyl; Isopropyl; Phenyl; o-, m- oder p-Tolyl; o-, m- oder p-Chlorphenyl; o-, m-oder p-Fluorphenyl; o-, m- oder p-Methoxyphenyl oder 2,3-, 2,4-, 2,5-, 2,6-, 3,4- oder 3,5-Dimethoxyphenyl bedeutet;
$R^2$ Wasserstoff; Phenyl; o-, m- oder p-Tolyl; o-, m- oder p-Chlorphenyl; o-, m- oder p-Fluorphenyl; o-, m- oder p-Methoxyphenyl; 2,3-, 2,4-, 2,5-, 2,6-, 3,4 oder 3,5-Dimethoxyphenyl; Phenoxy; o-, m- oder p-Tolyloxy; o-, m- oder p-Chlorphenoxy; o-, m- oder p-Fluorphenoxy; o-, m- oder p-Methoxyphenoxy; 2,3-, 2,4-, 2,5-, 2,6-, 3,4- oder 3,5-Dimethoxyphenoxy; 2-, 3- oder 4-Benzylphenoxy; 2-, 3- oder 4-Phenethyloxy; 2-, 3- oder 4-Styryloxy; Benzoyl; o-, m- oder p-Toluoyl; o-, m- oder p-Chlorbenzoyl; o-, m- oder p-Fluorbenzoyl; o-, m- oder p-Methoxybenzoyl; 2,3-, 2,4-, 2,5-, 2,6-, 3,4- oder 3,5-Dimethoxybenzoyl; Hydroxy oder Methoxy bedeutet;
$R^1$ und $R^2$ zusammen für Benzyliden; o-, m- oder p-Methylbenzyliden; o-, m- oder p-Methoxybenzyliden, 2,3-, 2,4-, 2,5-, 2,6-, 3,4- oder 3,5-Dimethoxybenzyliden oder 2-Phenyl-1-cyclopropyl stehen;
$R^6$ Wasserstoff oder Methyl bedeutet;
$R^7$ Wasserstoff; Methyl; Cyano; Benzoyl; o-, m- oder p-Methoxybenzoyl; o-, m- oder p-Chlorbenzoyl; 2,3-, 2,4-, 2,5-, 2,6-, 3,4- oder 3,5-Dimethoxybenzoyl; Acetyl; Methoxycarbonyl oder Ethoxycarbonyl bedeutet;
$R^8$ Cyano; Formyl; Benzoyl; o-, m- oder p-Methoxybenzoyl; o-, m- oder p-Chlorbenzoyl; 2,3-, 2,4-, 2,5-, 2,6-, 3,4-oder 3,5-Dimethoxybenzoyl; Acetyl; Methoxycarbonyl; Ethoxycarbonyl oder einen Rest der Formel

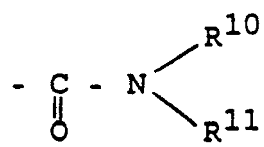

bedeutet;
$R^9$ Methyl; Ethyl; Benzyl oder Benzhydryl bedeutet;
$R^{10}$ und $R^{11}$ gleich oder verschieden sind und Wasserstoff; $(C_1$-$C_6)$-Alkyl; Phenyl oder Phenyl-$(C_1$-$C_4)$-alkyl, worin der Phenylrest jeweils durch einen oder zwei gleiche oder verschiedene Reste aus der Reihe Methyl, Methoxy, Fluor und Chlor, drei Methoxy, oder ein Methylendioxy substituiert sein kann; Cyclopentyl oder Cyclohexyl bedeutet; oder
$R^{10}$ und $R^{11}$ zusammen mit dem diese tragenden N-Atom einen 5-bis 7-gliedrigen heterocyclischen Ring bilden, der als Ringatome 2-6 Kohlenstoffatome und gegebenenfalls 1 oder 2 weitere gleiche oder verschiedene Heteroatome aus der Reihe Sauerstoff, Schwefel und Stickstoff enthält und der gegebenenfalls durch Methyl, Phenyl, Phenyl-$(C_1$-$C_4)$-alkyl, Benzoyl, Pyridyl oder Pyrimidinyl monosubstituiert ist, worin Phenyl, Phenylalkyl und Benzoyl ihrerseits jeweils im Phenylrest durch ein oder zwei gleiche oder verschiedene Reste aus der Reihe Methyl, Methoxy, Fluor, Chlor, Cyano und Nitro, oder drei Methoxy substituiert sein können;
X Sauerstoff bedeutet;
m = 0, 1, 2, 3, 4 oder 5 ist und
n = 0 oder 1 ist,
sowie deren physiologisch verträgliche Salze.

4. Verfahren gemäß einem oder mehreren der Ansprüche 1 bis 3, dadurch gekennzeichnet, daß man eine Verbindung der Formel I herstellt, in welcher
$R^2$ Wasserstoff bedeutet;
$R^6$ Wasserstoff bedeutet;
$R^8$ Wasserstoff bedeutet;
X Sauerstoff bedeutet;
m = 1, 2, 3 oder 4 ist und
n = 0 oder 1 ist,
sowie deren physiologisch verträgliche Salze.

5. Verfahren zur Herstellung einer pharmazeutischen Zubereitung enthaltend eine Verbindung gemäß einem der Ansprüche 1 bis 4, dadurch gekennzeichnet, daß man den Wirkstoff zusammen mit einem physiologisch annehmbaren Träger und gegebenenfalls weiteren Zusatz-, Hilfs-und/oder Konservierungsstoffen in eine geeignete Darreichungsform bringt.